# FASCICULE DE BREVET EUROPEEN

(11) **EP 0 880 538 B1**
(45) Date de publication et mention de la délivrance du brevet: **15.05.2002**
(21) Numéro de dépôt: 97903426.1
(22) Date de dépôt: 07.02.1997
(51) Int. Cl.: C07H 15/04, B01F 17/56

(54) **PROCEDE DE PREPARATION D'AGENTS TENSIOACTIFS**
VERFAHREN ZUR HERSTELLUNG VON TENSIDEN
METHOD FOR PREPARING SURFACE-ACTIVE AGENTS

(30) Priorité: 08.02.1996 FR 9601542
(43) Date de publication de la demande: 02.12.1998
(73) Titulaire: AGRO INDUSTRIE RECHERCHES ET DEVELOPPEMENTS (A.R.D.), 51110 Pomacle (FR)
(72) Inventeur: BERTHO, Jean-Noel, F-51100 Reims (FR); MATHALY, Philippe, F-51100 Reims (FR); DUBOIS, Véronique, F-51100 Reims (FR); de BAYNAST de SEPTFONTAINES, Régis, F-78000 Versailles (FR)
(74) Mandataire: Eidelsberg, Victor Albert
(86) Numéro de dépôt international: FR9700249
(87) Numéro de publication internationale: WO9729115

(56) Documents cités:
- WO-A-92/07098
- FR-A- 2 580 669
- US-A- 4 070 232
- US-A- 4 223 129
- DATABASE WPI Section Ch, Week 7843 Derwent Publications Ltd., London, GB; Class D17, AN 78-77886A XP002036876 & SU 583 137 A (AS KIRG ORG CHEM) , 12.Januar 1978

## Description

La présente invention se rapporte aux procédés de préparation de mélanges de glycosides d'alkyle, utiles en tant qu'agents tensioactifs.

Le greffage de groupes alkyle sur des glucides conduit à des agents tensioactifs dont les propriétés de surface sont très intéressantes et dont la biodégradabilité est généralement bonne (R.D. Swisher, "Surfactant biodégradation", Marcel Dekker, Inc. NEW YORK, 1987).

La réaction la plus couramment rencontrée constitue la glycosylation d'alcools gras par des sucres réducteurs ou des glycosides intermédiaires. Celle-ci est réalisée en présence d'un catalyseur acide et les glycosides d'alkyle obtenus sont stables dans un large domaine de pH, notamment neutre et basique. Le substrat le plus souvent utilisé comme sucre réducteur est le glucose. La glycosylation réalisée à partir de ce sucre conduit à des mélanges de glucosides et de polyglucosides d'alkyle qui sont connus pour leurs propriétés tensioactives. Ceux-ci présentent désormais de nombreuses applications dans des domaines aussi variés que la détergence, les industries chimiques et parachimiques, ou encore dans le domaine médical et la biologie (voir par exemple WO 93 07160, WO 93 07249, DE 42 12 080 A1, US 4 987 225). Pour la préparation de ces glucosides d'alkyle, il est nécessaire de disposer de grandes quantités de glucose. L'obtention de ce glucose à partir, par exemple, de la farine de mais nécessite de nombreuses étapes. Il est en effet indispensable d'éliminer les protéines et de purifier le glucose notamment par des étapes de filtration et de chromatographie sur résines échangeuses d'ions.

L'inconvénient majeur de cette approche réside dans le coût élevé de la matière première lié notamment à l'utilisation de matière agricole noble telle que la farine et aux nombreuses étapes de purification du glucose. Tout ceci limite naturellement les applications potentielles de ces procédés. En outre, la réaction de glycosylation du glucose ou autres hexoses s'effectue à haute température (100 à 150°C), ce qui donne une coloration des produits.

La réaction de glycosylation de l'amidon s'effectue également à haute température (100 à 400°C) (US 4, 223, 129).

Le brevet américain 4,070,232 décrit un procédé qui consiste à pre-hydrolyser des plantes vasculaires en milieu acide à chaud et à séparer une phase liquide contenant des pentoses et hexoses d'une phase fibreuse.Les sucres doivent être purifiés avant d'être valorisés éventuellement en tant qu'édulcorant, substrats pour l'alimentation animale, d'humidifiants, de tensio-actifs, de polyols, agents séquestrant et d'émulsifiants.

L'invention vise un procédé de préparation d'agents tensioactifs caractérisé en ce qu'il consiste à mettre de la bagasse, des sous-produits du maïs, des sous-produits du sorgho, des sous-produits de l'orge, des sous produits du riz, des fruits, de la pulpe de chicorée, des tubercules, du cynara en contact pendant au moins 5 secondes avec un agent d'hydrolyse choisi parmi une solution aqueuse acide entre 20 et 150°C et une composition enzymatique d'hydrolyse d'une matière végétale entre 20 et 90 °C pour obtenir un sirop de sucres si cela est nécessaire, à débarrasser le sirop de sucres d'un marc solide et à mettre le sirop de sucres exempt de marc en contact avec un alcool ayant de 4 à 22 atomes de carbone à une température comprise entre 20 et 150°C, et de préférence entre 30 et 110°C, jusqu'à obtention d'une solution de glycosides tensioactifs, et à séparer les glycosides tensioactifs de cette solution.

Ce procédé présente l'avantage d'utiliser des sucres actuellement mal valorisés tels que les pentoses et les acides uroniques provenant respectivement de l'hydrolyse de l'hémicellulose et de la pectine, de travailler à partir de mélanges de ces sucres et par conséquent d'éviter des étapes de purification comme la cristallisation. De plus, nous avons constaté avec surprise que les oligomères sont greffés avec un alcool gras ce qui permet notamment d'améliorer les rendements de greffage et de valoriser complètement le jus sucré contrairement au brevet US 4,070,232.

Le sirop est constitué notamment de pentoses, en particulier de D-xylose et de L-arabinose et parfois d'acides uroniques tel que l'acide D-galacturonique. D'une manière surprenante, nous avons mis en évidence que les pentoses tels que le D-xylose et le L-arabinose et l'acide D-galacturonique présentaient l'avantage d'être plus réactifs lors de la glycosylation. Cette propriété permet un greffage dans des conditions ménagées évitant notamment toute dégradation des réactifs ou des produits de la réaction. Contrairement à la préparation des glucosides et polyglucosides d'alkyle qui nécessite des températures généralement supérieures à 120 °C (voir DE 42 12 080, WO 93 07160), la synthèse des glycosides d'alkyle selon la présente invention peut être réalisée à une température inférieure à 100°C. De plus, à température égale, nous avons montré que la glycosidation de l'arabinose, du xylose et de l'acide galacturonique s'effectue plus rapidement que la glycosidation du glucose. A 80°C, à partir de sucres cristallisés, dans les mêmes conditions de réaction et au bout d'une heure de réaction, 100% de l'acide D-galacturonique et 87% du L-arabinose ont été consommés tandis qu'il reste encore 97% de D-glucose résiduel.

De plus, nous avons pu mettre en évidence en travaillant cette fois à partir d'un mélange de D-glucose et de L-arabinose une synergie entre ces deux sucres. Le D-glucose réagit en effet plus vite en présence d'arabinose que seul dans le milieu réactionnel. A 80°C, à partir de sucres cristallisés, dans les mêmes conditions de réaction et au bout de 2 heures de réaction, il reste 25% et 40% du D-glucose de départ lorsqu'il est traité respectivement en mélange avec 90% et 40% de L-arabinose tandis qu'il reste 60% du D-glucose de départ lorsqu'il est utilisé pur.
Ces résultats confortent notre approche qui consiste à travailler à partir de mélange de sucres contenant des pentoses. Les durées de réaction sont ainsi considérablement diminuées.

Selon la présente invention, les rendements en tensioactifs à partir de la bagasse, des sous-produits du maïs, des sous-produits du sorgho, des sous-produits de l'orge, des sous produits du riz, des fruits, de la pulpe de chicorée, des tubercules, du cynara sont proches de ceux obtenus théoriquement à partir de ces végétaux en tenant compte de la composition en hémicellulose et pectines de la matière première.
Ainsi, dans le gros son de maïs pour une composition théorique de 55 % en sucres glycosidables par rapport à la matière sèche totale, nous greffons après purification 49 % de la matière sèche de départ soit 90 % du potentiel.

On a en outre la possibilité d'obtenir séparément des agents tensioactifs dérivés de sucres neutres non-chargés (pentoses et hexoses) et des agents tensioactifs dérivés de sucres anioniques (acide galacturonique).

De plus, la réaction de glycosylation peut être réalisée sans apport de catalyseurs acides supplémentaires ; l'acidité du sirop permet en effet la catalyse de la réaction.

L'invention vise à obtenir des agents tensioactifs à partir d'une matière première particulièrement bon marché par des stades opératoires, simples et peu nombreux, qui ne nécessitent pas l'application de températures élevées induisant une coloration.

Suivant l'invention, on utilise de la bagasse, des sous-produits du maïs, des sous-produits du sorgho, des sous-produits de l'orge, des sous produits du riz, des fruits, de la pulpe de chicorée, des tubercules, du cynara comme matières premières pour la préparation d'agents tensioactifs.

On entend par sous-produits du maïs et du sorgho, la totalité de la plante et/ou les organes constitutifs (rafles, spathes, tiges, feuilles) qui peuvent être récoltés directement ou provenir de déchets après séparation des grains.

On peut également utiliser de la fibre de maïs ou de riz. On entend par fibre de maïs les composés obtenus au cours d'un procédé de fractionnement, visant notamment à produire de l'amidon.

Le son est obtenu après broyage au moulin du grain. Il est lui-même composé d'hémicellulose constitué de polymères de xylose et arabinose, et de cellulose.

On peut également utiliser les fibres d'orge issues de brasserie. Ces fibres correspondent aux marcs obtenus après pressage du malt d'orge saccharifié.

Suivant l'invention, on entend par fruit principalement les fruits dits complexes comme la pomme, ou les fruits charnus comme les agrumes.

On peut également utiliser les sous produits de l'industrie du jus de fruit comme le marc de pomme, de poire, ou de citrus.

Ces différents marcs sont composés d'hémicelluloses constituées de monomères d'arabinose et de galactose et de pectines constituées essentiellement de monomères d'acide galacturonique.

Suivant l'invention, on peut utiliser des tubercules comme la pomme de terre ou l'oignon.

On peut également utiliser les pelures d'oignon ou de pomme de terre ainsi que les produits de première transformation comme la purée de pomme de terre, les frites.
Les pelures de pomme de terre ou d'oignon sont composées d'hémicelluloses constituées de monomères d'arabinose et de xylose et de pectines constituées de monomères d'acide galacturonique.

Suivant l'invention on utilise également le cynara, on entend par cynara toute plante appartenant à la famille des cynara comme l'artichaut (cynara scolymus) ou toute plante hybride issue d'un croisement entre un cynara et un carduus ou un cirsium (chardon).

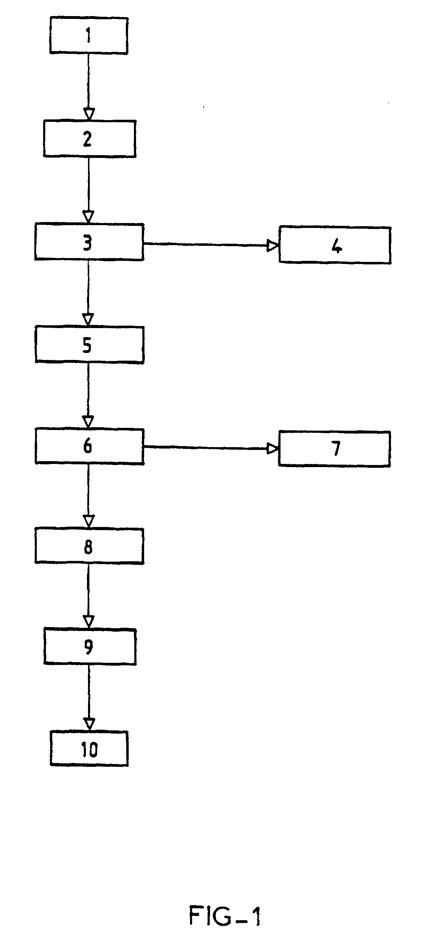

**Dans le cas où le substrat de départ ne contient pas de pectines** (figure 1), le premier stade du procédé suivant l'invention consiste à mettre de la bagasse, des sous-produits du maïs, des sous-produits du sorgho, des sous-produits de l'orge, des sous-produits de riz, en contact avec une solution aqueuse acide (2). On extrait, solubilise et hydrolyse ainsi en milieu aqueux en présence d'acide ou d'enzymes, l'hémicellulose de la bagasse, des sous-produits du maïs, des sous-produits du sorgho, des sous-produits de l'orge , des sous-produits de riz, (1). L'acide utilisé pouvant être l'acide chlorhydrique, l'acide bromhydrique, l'acide sulfurique, l'acide phosphorique, un acide sulfonique tel que l'acide benzènesulfonique, l'acide paratoluènesulfonique, l'acide camphresulfonique, l'acide sulfosuccinique ou un sulfosuccinate d'alkyle tel que le sulfosuccinate de décyle ou de lauryle, les acides perhalohydriques, tels que par exemple l'acide perchlorique .

On effectue généralement la mise en milieu acide pendant 5 à 90 minutes dans un mélangeur simple. Mais on peut ramener la durée de contact à quelques secondes en opérant dans un dispositif permettant d'effectuer des mélanges en ligne, connu sous le nom de "jet cooker".
On peut aussi effectuer le premier stade par hydrolyse enzymatique.Les enzymes utilisées pouvant être un complexe multienzymatique contenant des hémicellulases, des cellulases, des β glucanases, des protéases, et des estérases.
L'hydrolyse enzymatique s'effectue en milieu aqueux pendant au moins une heure à une température comprise entre 20 et 90°C.

Le deuxième stade du procédé suivant l'invention (3) consiste à débarrasser le sirop de sucres d'un marc solide (4). On effectue avantageusement cette opération par pressage sous une pression de l'ordre de 1.10⁵ à 500.10⁵ Pascals et de préférence de l'ordre de 5.10⁵ à 60.10⁵ Pascals au moyen d'une presse à vis, de filtres presses, ou avec tout autre équipement permettant d'exprimer un jus à partir de marc humide. On obtient ainsi un jus de sucres (5).

On peut éventuellement permuter les jus de sucres sous forme acide par échange d'ions sur une résine échangeuse de cations ou par électodialyse (9).

On peut également concentrer le sirop de sucres par évaporation jusqu'à 30 à 95 % et plus particulièrement 60 à 80 % de matière sèche (9). Le sirop de sucres débarrassé du marc solide présente une pureté en sucres généralement comprise entre 50 et 95 % par rapport à la matière sèche.

Le troisième stade du procédé suivant l'invention consiste à mettre le sirop de sucres débarrassé du marc en contact avec un alcool ayant de 4 à 22 atomes de carbone. L'alcool répond à la formule R¹OH, R¹ étant un radical linéaire ou ramifié alkyle de 6 à 22 atomes de carbone, hydrocarboné ayant de 1 à 4 insaturations éthyléniques et de 4 à 22 atomes de carbone ou l'un de ces radicaux substitués par 1 à 3 substituants sur des atomes de carbone différents choisis parmi hydroxy, halogène et trifluorométhyle. On préfère tout particulièrement les alcools gras ayant de 8 à 16 atomes de carbone ; et notamment, les mélanges d'octanol et de décanol, les mélanges de décanol et de dodécanol, les mélanges de dodécanol et de tétradécanol et les mélanges de dodécanol, de tétradécanol et d'hexadécanol. On utilise en général de 1 à 4 équivalents d'alcool par rapport aux sucres. On préfère cependant dans un premier temps réaliser la glycosylation d'un alcool court par les sucres du sirop. On réalise par la suite une transglycosylation par des alcools gras de formule R¹OH. L'alcool court répond à la formule R²OH, R² étant un radical linéaire alkyle ayant de 1 à 5 atomes de carbone. On préfère effectuer cette réaction de glycosylation en l'absence totale de solvants, mais on peut le cas échéant utiliser un solvant tel qu'un éther oxyde tel que le tétrahydrofurane, l'oxyde diéthylique, le 1,4-dioxane, l'oxyde isopropylique, le méthyl-tertiobutyl éther, l'éthyl-tertiobutyl éther ou le diglyme, un hydrocarbure halogéné tel que le chlorure de méthylène, le chloroforme, le 1,2-dichloroéthane, un solvant nitré tel que le nitrométhane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène. On préfère effectuer cette réaction entre 30 et 110°C pour prévenir le plus possible la formation de produit coloré, mais il est possible de l'effectuer jusqu'à 150°C. Dans le cas où le jus de sucres a été complètement déminéralisé, il est nécessaire d'ajouter au milieu réactionnel 0,1 à 5 % en masse par rapport à la matière sèche du sirop d'un catalyseur acide. Cet acide pouvant être l'acide chlorydrique, l'acide sulfurique, l'acide phosphorique, un acide sulfonique tel que l'acide benzènesulfonique, l'acide paratoluènesulfonique, l'acide camphresulfonique, l'acide sulfosuccinique ou un sulfosuccinate d'alkyle tel que le sulfosuccinate de décyle ou de lauryle, les acides perhalohydriques, tels que par exemple l'acide perchlorique. Cette catalyse acide peut être également effectuée par 0,05 à 6 équivalents pondéraux d'une résine sulfonique sous sa forme H⁺, ou d'une résine acide. On préfère éviter la déminéralisation totale du jus de presse et par conséquent ne pas rajouter de catalyseur acide.

Pour recueillir le mélange de glycosides d'alkyle, le procédé consiste :
- à éliminer le solvant de réaction, s'il est présent,
- à neutraliser l'acidité du sirop de départ ou le catalyseur acide, puis éventuellement à filtrer le sel obtenu. On effectue la neutralisation, par exemple, par un hydrogénocarbonate ou un carbonate de métal alcalin ou alcalino-terreux, notamment l'hydrogénocarbonate de sodium, par un hydroxyde de métal alcalin ou alcalino-terreux, notamment la soude.
   on obtient alors un tensio-actif au sens de l'innovation sous la forme d'une base auto émulsionnante.
- mais si cela est souhaité, on peut purifier le produit :
   - soit par évaporation de l'alcool gras en excès sous vide compris entre 0,001 et 10 mbars à une température comprise entre 60 et 250°C,
   - soit par chromatographie sur colonne de gel de silice, d'alumine, de charbon actif ou sur résine échangeuse d'ions,
   - soit par cristallisation dans un solvant ou un mélange de solvants appropriés choisis parmi les éthers oxydes tels que le tétrahydrofurane, l'oxyde diéthylique, le 1,4-dioxane, l'oxyde isopropylique, le méthyl-tertiobutyl éther, l'éthyl-tertiobutyl éther ou le diglyme, un hydrocarbure halogéné tel que le chlorure de méthylène, le chloroforme, le 1,2-dichloroéthane, un solvant nitré tel que le nitrométhane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène,
   - soit par extractions sélectives par des solvants non miscibles à l'eau,
      Le produit isolé présente alors un pourcentage d'alcool gras résiduel compris entre 0 et 5% et de préférence entre 0 et 1%.
   - éventuellement solubiliser le tensioactif dans l'eau pour obtenir une composition tensioactive présentant une matière sèche de 20 à 80 %,
   - si besoin, décolorer cette solution en ajoutant à une température comprise entre 15 et 100 °C, 0,05 à 10 % et de préférence de 0,5 à 3 % de peroxyde d'hydrogène, de peroxodisulfates de métaux alcalins ou alcalino-terreux, de perborates, de persulfates, de perphosphates, de percarbonates, d'ozone ou encore de periodinates. On préfère le peroxyde d'hydrogène à 30 ou 50 %. L'agent de décoloration de la présente invention doit naturellement être compatible avec l'ensemble des ingrédients de la formulation finale et avec les applications des produits finis.

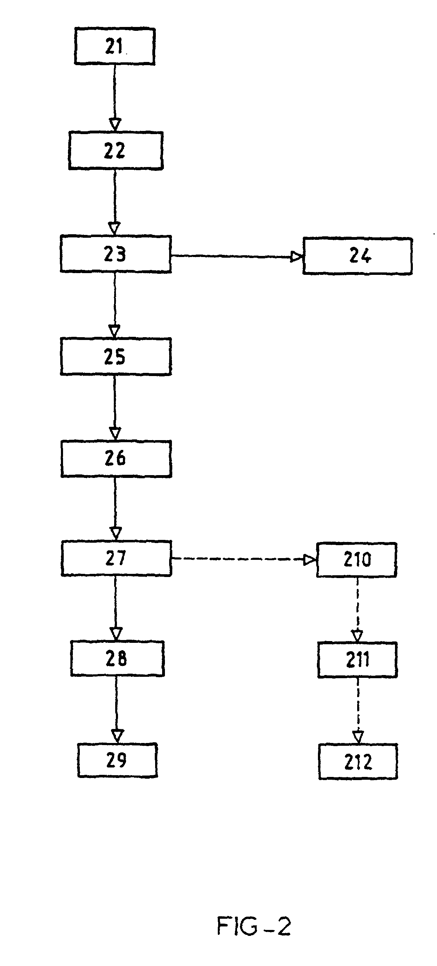

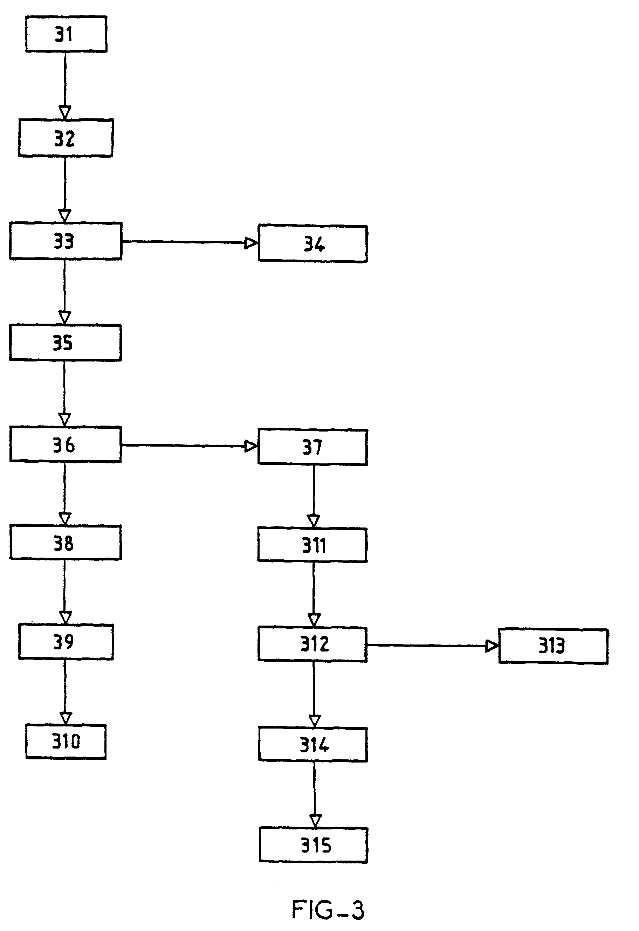

**Dans le cas où le substrat de départ contient des pectines** (figures 2 et 3), le premier stade du procédé suivant l'invention consiste à mettre des fruits, de la pulpe de chicorée, des tubercules, du cynara (21, 31) en contact avec une solution aqueuse acide (22, 32). On extrait, solubilise et hydrolyse ainsi en milieu aqueux en présence d'acide l'hémicellulose des fruits, de la pulpe de chicorée, des tubercules, du cynara. Pendant ce traitement les pectines des des fruits, de la pulpe de chicorée, des tubercules, du cynara sont solubilisées (22, 32). L'acide utilisé pouvant être l'acide chlorhydrique, l'acide bromhydrique, l'acide sulfurique, l'acide phosphorique, un acide sulfonique tel que l'acide benzènesulfonique, l'acide paratoluènesulfonique, l'acide camphresulfonique, l'acide sulfosuccinique ou un sulfosuccinate d'alkyle tel que le sulfosuccinate de décyle ou de lauryle, les acides perhalohydriques, tels que par exemple l'acide perchlorique.

On effectue généralement cette mise en contact pendant 5 à 90 minutes dans un mélangeur simple. Mais on peut ramener la durée de contact à quelques secondes en opérant dans un dispositif permettant d'effectuer des mélanges en ligne, connu sous le nom de "jet cooker". On peut aussi effectuer le premier stade par hydrolyse enzymatique.Les enzymes utilisées pouvant être un complexe multienzymatique pouvant contenir des hémicellulases, des cellulases, des β glucanases, des protéases, et des estérases, des pectinases.
L'hydrolyse enzymatique s'effectue en milieu aqueux pendant au moins une heure à une température comprise entre 20 et 90°C.

Le deuxième stade du procédé suivant l'invention (23,33) consiste à débarrasser le sirop de sucres d'un marc solide (24,34). On effectue avantageusement cette opération par pressage sous une pression de l'ordre de 1.10⁵ à 500.10⁵ Pascals et de préférence de l'ordre de 5.10⁵ à 60.10⁵ Pascals au moyen d'une presse à vis, de filtres presses de décanteuses ou avec tout autre équipement permettant d'exprimer un jus à partir de marc humide. On obtient ainsi un jus de sucres (25,35).

On peut éventuellement réaliser une hydrolyse enzymatique complémentaire du jus purifié (27) afin d'obtenir un jus de sucres hydrolysé enzymatiquement. Cette opération consiste à mettre en contact le jus issu de la purification (26) avec des pectinases pendant au moins 5 minutes, à une température comprise entre 10 et 60 °C et à un pH compris entre 3 et 6 (27).

On peut aussi permuter l'hydrolysat obtenu sous forme acide par passage sur une résine échangeuse de cation ou par électrodialyse (28).

On peut également concentrer le sirop de sucres par évaporation jusqu'à 30 à 95 % et plus particulièrement 60 à 80 % de matière sèche (29). Le sirop de sucres débarrassé du marc solide présente une pureté en sucres généralement comprise entre 50 et 95 % par rapport à la matière sèche.

Le troisième stade du procédé suivant l'invention consiste à mettre le sirop de sucres débarrassé du marc en contact avec un alcool ayant de 4 à 22 atomes de carbone selon le protocole décrit pour le traitement des matières premières ne contenant pas de pectines.

Pour recueillir le mélange de glycosides d'alkyle, le procédé consiste :
- à éliminer le solvant de réaction, s'il est présent,
- à neutraliser l'acidité du sirop de départ ou le catalyseur acide, puis éventuellement à filtrer le sel obtenu. On effectue la neutralisation, par exemple, par un hydrogénocarbonate ou un carbonate de métal alcalin ou alcalino-terreux, notamment l'hydrogénocarbonate de sodium, par un hydroxyde de métal alcalin ou aicalino-terreux, notamment la soude,
- éventuellement à saponifier les (alkyl-D-galactoside) uronates d'alkyle formés au cours du greffage. Pour cette opération on peut utiliser un hydroxyde de métal alcalin ou alcalino-terreux, notamment la soude.
- et si cela est souhaité, on peut purifier le produit :
   - soit par évaporation de l'alcool gras en excès sous vide compris entre 0,001 et 10 mbars à une température comprise entre 60 et 250°C,
   - soit par chromatographie sur colonne de gel de silice, d'alumine, de charbon actif ou sur résine échangeuse d'ions,
   - soit par cristallisation dans un solvant ou un mélange de solvants appropriés choisis parmi les éthers oxydes tels que le tétrahydrofurane, l'oxyde diéthylique, le 1,4-dioxane, l'oxyde isopropylique, le méthyl-tertiobutyl éther, l'éthyl-certiobutyl éther ou le diglyme, un hydrocarbure halogéné tel que le chlorure de méthylène, le chloroforme, le 1,2-dichloroéthane, un solvant nitré tel que le nitrométhane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène,
   - soit par extractions sélectives par des solvants non miscibles à l'eau,
      Le produit isolé présente alors un pourcentage d'alcool gras résiduel compris entre 0 et 5% et de préférence entre 0 et 1%.
   - éventuellement solubiliser le tensioactif dans l'eau pour obtenir une composition tensioactive présentant une matière sèche de 20 à 80 %,
   - si besoin, décolorer cette solution en ajoutant à une température comprise entre 15 et 100 °C, 0,05 à 10 % et de préférence de 0,5 à 3 % de peroxyde d'hydrogène, de peroxodisulfates de métaux alcalins ou alcalino-terreux, de perborates, de persulfates, de perphosphates, de percarbonates, d'ozone ou encore de periodinates. On préfère le peroxyde d'hydrogène à 30 ou 50 %. L'agent de décoloration de la présente invention doit naturellement être compatible avec l'ensemble des ingrédients de la formulation finale et avec les applications des produits finis.

**Dans le cas où le substrat de départ contient des pectines une première variante du procédé** consiste après avoir effectué l'hydrolyse enzymatique complémentaire qui donne un jus de sucres hydrolysés enzymatiquement (figure 2 (22)), à le séparer en un jus de sucres neutres et en un jus de sucres anioniques. Cette opération (27) peut être réalisée par électrodialyse ou en fixant puis éluant les sucres anioniques sur une résine d'échange d'ions anionique.

On peut aussi permuter le jus de sucres anioniques obtenu sous forme acide par passage sur une résine échangeuse de cation ou par électrodialyse (211).

On peut également concentrer le sirop de sucres anioniques par évaporation jusqu'à 30 à 95 % et plus particulièrement 60 à 80 % de matière sèche (212). Le sirop de sucres anioniques débarrassé du marc solide présente une pureté en sucres anioniques généralement comprise entre 50 et 95 % par rapport à la matière sèche.

Le troisième stade du procédé suivant l'invention consiste à mettre le sirop de sucres anioniques débarrassé du marc en contact avec un alcool ayant de 4 à 22 atomes de carbone selon le protocole décrit pour le traitement des matières premières ne contenant pas de pectines.

Pour recueillir le mélange de glycosides d'alkyle, le procédé consiste :
- à éliminer le solvant de réaction, s'il est présent,
- à neutraliser l'acidité du sirop de départ ou le catalyseur acide, puis éventuellement à filtrer le sel obtenu. On effectue la neutralisation, par exemple, par un hydrogénocarbonate ou un carbonate de métal alcalin ou alcalino-terreux, notamment l'hydrogénocarbonate de sodium, par un hydroxyde de métal alcalin ou alcalino-terreux, notamment la soude,
- éventuellement à saponifier les (alkyl-D-galactoside) uronates d'alkyle formés au cours du greffage. Pour cette opération on peut utiliser un hydroxyde de métal alcalin ou alcalino-terreux, notamment la soude.
- et si cela est souhaité, on peut purifier le produit :

- soit par évaporation de l'alcool gras en excès sous vide compris entre 0,001 et 10 mbars à une température comprise entre 60 et 250°C,
- soit par chromatographie sur colonne de gel de silice, d'alumine, de charbon actif ou sur résine échangeuse d'ions,
- soit par cristallisation dans un solvant ou un mélange de solvants appropriés choisis parmi les éthers oxydes tels que le tétrahydrofurane, l'oxyde diéthylique, le 1,4-dioxane, l'oxyde isopropylique, le méthyl-tertiobutyl éther, l'éthyl-tertiobutyl éther ou le diglyme, un hydrocarbure halogéné tel que le chlorure de méthylène, le chloroforme, le 1,2-dichloroéthane, un solvant nitré tel que le nitrométhane, le nitro-2- propane, un solvant de la famille des amides tel que le N-méthylformamide, le N,N-diméthylformamide, le N,N-diméthylacétamide, la N-méthyl-2-pyrrolidone, un nitrile tel que l'acétonitrile, un alcane comme l'hexane, l'heptane, ou l'octane, ou un solvant aromatique tel que le toluène ou le xylène,
- soit par extractions sélectives par des solvants non miscibles à l'eau,
   Le produit isolé présente alors un pourcentage d'alcool gras résiduel compris entre 0 et 5% et de préférence entre 0 et 1%.
- éventuellement solubiliser le tensioactif dans l'eau pour obtenir une composition tensioactive présentant une matière sèche de 20 à 80 %,
- si besoin, décolorer cette solution en ajoutant à une température comprise entre 15 et 100 °C, 0,05 à 10 % et de préférence de 0,5 à 3 % de peroxyde d'hydrogène, de peroxodisulfates de métaux alcalins ou alcalino-terreux, de perborates, de persulfates, de perphosphates, de percarbonates, d'ozone ou encore de periodinates. On préfère le peroxyde d'hydrogène à 30 ou 50 %. L'agent de décoloration de la présente invention doit naturellement être compatible avec l'ensemble des ingrédients de la formulation finale et avec les applications des produits finis.

On peut éventuellement permuter les jus de sucres neutres par échange d'ions sur une résine échangeuse de cations ou par électodialyse (28).

On peut également concentrer le sirop de sucres neutres par évaporation jusqu'à 30 à 95 % et plus particulièrement 60 à 80 % de matière sèche (9). Le sirop de sucres neutres débarrassé du marc solide présente une pureté en sucres neutres généralement comprise entre 50 et 95 % par rapport à la matière sèche.

Le troisième stade du procédé suivant l'invention consiste à mettre le sirop de sucres neutres débarrassé du marc en contact avec un alcool ayant de 4 à 22 atomes de carbone selon le protocole décrit pour le traitement des matières premières ne contenant pas de pectines.

Pour recueillir le mélange de glycosides d'alkyle, le procédé consiste à réaliser le même protocole que pour le traitement des matières premières ne contenant pas de pectines.

**Dans le cas où le substrat de départ contient des pectines une seconde variante du procédé** consiste, après avoir mis en contact des fruits, de la pulpe de chicorée, des tubercules, du cynara et une solution aqueuse acide (figure 3 (32)),
- à débarrasser (33) le sirop de sucres d'un marc solide (34) afin d'obtenir un jus de sucres (35),
- à mettre en contact le jus de sucres (35) avec des sels chélatants (36) de manière à obtenir un jus de sucres non chargés et des boues (37). On peut notamment utiliser des cations métalliques tels que l'hydroxyde d'aluminium, le sulfate d'aluminium, des cations alcalino-terreux tels que les hydroxydes de magnésium, de calcium, de strontium ou de baryum, les sulfates de magnésium, de calcium, de strontium ou de baryum, les chlorures de magnésium, de calcium, de strontium ou de baryum.
- éventuellement à permuter le jus de sucres non chargés sous forme acide par passage sur une résine échangeuse de cation ou par électrodialyse (39),
- éventuellement à concentrer le jus de sucres non chargés par évaporation jusqu'à 30 à 95 % et plus particulièrement 60 à 80 % de matière sèche (310),
- à mettre le jus de sucres non chargés avec un alcool ayant de 4 à 22 atomes de carbone selon le protocole décrit pour le traitement des matières premières ne contenant pas de pectines.
- à recueillir le mélange de glycosides d'alkyle selon le protocole décrit pour le traitement des matières premières ne contenant pas de pectines.
- éventuellement à mettre les boues (37) en contact avec une ou plusieurs enzymes (311) pour obtenir des boues hydrolysées,
- à séparer les boues (312) en un liquide et un solide (313),
- éventuellement à permuter le liquide sous forme acide par passage sur une résine échangeuse de cation ou par électrodialyse (314),
- éventuellement à concentrer le liquide par évaporation jusqu'à 30 à 95 % et plus particulièrement 60 à 80 % de matière sèche (310),
- à mettre le liquide en contact avec un alcool ayant de 4 à 22 atomes de carbone selon le protocole décrit pour le traitement des matières premières ne contenant pas de pectines.
- à recueillir le mélange de glycosides d'alkyle selon le protocole décrit pour le traitement des matières premières contenant des pectines.

### EN PRATIQUE :

**Dans le cas où le substrat de départ ne contient pas de pectines** (figure 1), le procédé consiste :
- à mélanger les sous produits agricoles (1) tels que de la bagasse, des sous-produits du maïs, des sous-produits du sorgho, des sous-produits de l'orge, les sous-produits du riz, à une solution aqueuse d'acide. On préfère l'acide sulfurique, l'acide chlorhydrique ou l'acide sulfosuccinique,
- à homogénéiser, puis à cuire le milieu à une température comprise entre 20 et 150 °C, plus particulièrement entre 80 et 140 °C, pendant une durée de 1 à 90 minutes (2)
   On peut aussi effectuer le premier stade par hydrolyse enzymatique.Les enzymes utilisées pouvant être un complexe multienzymatique contenant des hémicellulases, des cellulases, des β glucanases, des protéases, et des estérases.
   L'hydrolyse enzymatique s'effectue en milieu aqueux pendant au moins une heure à une température comprise entre 20 et 90°C.
- à presser le mélange à une pression de l'ordre de 1.10⁵ à 500.10⁵ Pascals et de préférence de l'ordre de 5.10⁵ à 60.10⁵ Pascals au moyen d'une presse à vis, de filtres presses, ou avec tout autre équipement permettant d'exprimer un jus à partir de marc humide. Cette opération (3) permet d'isoler un jus de sucres (5) et un marc (4),
- éventuellement à diluer les marcs (4) avec au moins trois fois leur volume d'eau et les presser sur une presse identique à celle utilisée lors du premier pressage. Cette opération n'est pas indispensable mais permet d'augmenter le rendement d'extraction en sucres.
- à rassembler les jus des presses (jus de sucres 5)
- éventuellement à recycler une partie ou l'ensemble du jus brut acide (5) pour l'hydrolyse de nouveaux sous produits tels que la bagasse, des sous-produits du maïs, des sous-produits de l'orge, des sous-produits du riz, et de préférence avec une quantité de jus brut acide similaire à la première dilution. Plusieurs recyclages à la suite sont possibles et permettent d'hydrolyser les sous produits tels que la bagasse, des sous-produits du maïs, des sous-produits de l'orge, des sous-produits du riz, sans apport d'eau supplémentaire, seule la quantité d'acide est ajustée,
- éventuellement à précipiter les impuretés contenues dans le jus de sucres en présence de sels chélatants (6). On peut notamment utiliser des cations métalliques tels que l'hydroxyde d'aluminium, le sulfate d'aluminium, des cations alcalino-terreux tels que les hydroxydes de magnésium, de calcium, de strontium ou de baryum, les sulfates de magnésium, de calcium, de strontium ou de baryum, les chlorures de magnésium, de calcium, de strontium ou de baryum.
- Si nécessaire à centrifuger le jus obtenu au moyen d'un équipement permettant d'obtenir des jus débarassés d'insolubles, tels qu'une décanteuse horizontale, un filtre presse, un décanteur statique, un filtre rotatif sous vide, des systèmes de filtration tangentielle, une centrifugeuse à assiettes auto-débourbeuse ou un filtre rotatif sous vide. On obtient alors un jus dépourvu d'insolubles et un culot (7),
- éventuellement à purifier la solution de sucres que l'on appelle jus, par déminéralisation, par chromatographie d'échange d'ions ou d'exclusion à basse pression, par électrodialyse ou encore par une combinaison des méthodes précédentes (8) tout en laissant, de préférence, pour ne pas à avoir à ajouter ensuite de l'acide servant de catalyseur, au moins 0,02 équivalent H⁺ par mole de sucres. En chromatographie, on réunit donc la fraction de sucres à une partie de la fraction de sels. Sur résine d'échange d'ions, on passe de préférence successivement sur une résine échangeuse de cations et sur une résine échangeuse d'anions, la quantité de résine échangeuse d'anions pouvant être inférieure d'au moins 0,02 équivalents H⁺ à la stoechiométrie.
- éventuellement à permuter les jus de sucres par échange d'ions sur une résine échangeuse de cations ou par électodialyse (9).
- si nécessaire à concentrer le sirop de sucres par évaporation jusqu'à 30 à 95 % et plus particulièrement 60 à 80 % de matière sèche (29, 212). Le sirop de sucres débarrassé du marc solide présente une pureté en sucres généralement comprise entre 50 et 95 % par rapport à la matière sèche.
- à effectuer la glycosylation par l'alcool,
- à recueillir le mélange de glycosides d'alkyle tensioactifs.

**Dans le cas où le substrat de départ contient des pectines** (figure 3), le procédé consiste :
- à mélanger les sous produits agricoles (31) tels que de les fruits, la pulpe de chicorée, les tubercules, le cynara à une solution aqueuse d'acide. On préfère l'acide sulfurique, l'acide chlorhydrique ou l'acide sulfosuccinique,
- à homogénéiser, puis à cuire le milieu à une température comprise entre 20 et 150 °C, plus particulièrement entre 80 et 140 °C, pendant une durée de 1 à 90 minutes (32).
   On peut aussi effectuer le premier stade par hydrolyse enzymatique.Les enzymes utilisées pouvant être un complexe multienzymatique contenant des hémicellulases, des cellulases, des β glucanases, des protéases, et des estérases, des pectinases.
   L'hydrolyse enzymatique s'effectue en milieu aqueux pendant au moins une heure à une température comprise entre 20 et 90°C.
- à presser le mélange à une pression de l'ordre de 1.10⁵ à 500.10⁵ Pascals et de préférence de l'ordre de 5.10⁵ à 60.10⁵ Pascals au moyen d'une presse à vis, de filtres presses de décanteuses ou avec tout autre équipement permettant d'exprimer un jus à partir de marc humide. Cette opération (33) permet d'isoler un jus de sucres (35) et un marc (34),
- éventuellement à diluer les marcs (34) avec au moins trois fois leur volume d'eau et les presser sur une presse identique à celle utilisée lors du premier pressage. Cette opération n'est pas indispensable mais permet d'augmenter le rendement d'extraction en sucres.
- à rassembler les jus des presses (jus de sucres 35)
- éventuellement à recycler une partie ou l'ensemble du jus brut acide (35) pour l'hydrolyse de nouveaux sous produits tels que les fruits, la pulpe de chicorée, les tubercules, le cynara, et de préférence avec une quantité de jus brut acide similaire à la première dilution. Plusieurs recyclages à la suite sont possibles et permettent d'hydrolyser les sous produits tels que les fruits, la pulpe de chicorée, les tubercules, le cynara, sans apport d'eau supplémentaire, seule la quantité d'acide est ajustée,
- éventuellement à purifier la solution de sucres que l'on appelle jus (35), par déminéralisation, par chromatographie d'échange d'ions ou d'exclusion à basse pression, par électrodialyse ou encore par une combinaison des méthodes précédentes (36) tout en laissant, de préférence, pour ne pas à avoir à ajouter ensuite de l'acide servant de catalyseur, au moins 0,02 équivalent H⁺ par mole de sucres. En chromatographie, on réunit donc la fraction de sucres à une partie de la fraction de sels. Sur résine d'échange d'ions, on passe de préférence successivement sur une résine échangeuse de cations et sur une résine échangeuse d'anions, la quantité de résine échangeuse d'anions pouvant être inférieure d'au moins 0,02 équivalents H⁺ à la stoechiométrie.
- éventuellement à permuter l'hydrolysat obtenu sous forme acide par passage sur une résine échangeuse de cation ou par électrodialyse (38).
- si nécessaire à concentrer le sirop de sucres par évaporation jusqu'à 30 à 95 % et plus particulièrement 60 à 80 % de matière sèche (39). Le sirop de sucres débarrassé du marc solide présente une pureté en sucres généralement comprise entre 50 et 95 % par rapport à la matière sèche.
- à effectuer la glycosylation par l'alcool.
- à recueillir les glycosides d'alkyle tensioactifs.

**Dans le cas où le substrat de départ contient des pectines selon une première variante** (figure 2), le procédé consiste :
- à mélanger les sous produits agricoles (21) tels que de les fruits, la pulpe de chicorée, les tubercules, le cynara à une solution aqueuse d'acide. On préfère l'acide sulfurique, l'acide chlorhydrique ou l'acide sulfosuccinique,
- à homogénéiser, puis à cuire le milieu à une température comprise entre 20 et 150 °C, plus particulièrement entre 80 et 140 °C, pendant une durée de 1 à 90 minutes (2)
   On peut aussi effectuer le premier stade par hydrolyse enzymatique.Les enzymes utilisées pouvant être un complexe multienzymatique contenant éventuellement des hémicellulases, des cellulases, des β glucanases, des protéases, des estérases et des pectinases.
   L'hydrolyse enzymatique s'effectue en milieu aqueux pendant au moins une heure à une température comprise entre 20 et 90°C.
- à presser le mélange à une pression de l'ordre de 1.10⁵ à 500.10⁵ Pascals et de préférence de l'ordre de 5.10⁵ à 60.10⁵ Pascals au moyen d'une presse à vis, de filtres presses ou avec tout autre équipement permettant d'exprimer un jus à partir de marc humide. Cette opération (23) permet d'isoler un jus de sucres (25) et un marc (24),
- éventuellement à diluer les marcs (24) avec au moins trois fois leur volume d'eau et les presser sur une presse identique à celle utilisée lors du premier pressage. Cette opération n'est pas indispensable mais permet d'augmenter le rendement d'extraction en sucres.
- à rassembler les jus des presses (jus de sucres 25)
- éventuellement à recycler une partie ou l'ensemble du jus brut acide (25) pour l'hydrolyse de nouveaux sous produits tels que les fruits, la pulpe de chicorée, les tubercules, le cynara, et de. préférence avec une quantité de jus brut acide similaire à la première dilution. Plusieurs recyclages à la suite sont possibles et permettent d'hydrolyser les sous produits tels que les fruits, la pulpe de chicorée, les tubercules, le cynara, sans apport d'eau supplémentaire, seule la quantité d'acide est ajustée,
- éventuellement à purifier la solution de sucres que l'on appelle jus (25), par déminéralisation, par chromatographie d'echange d'ions ou d'exclusion à basse pression, par électrodialyse ou encore par une combinaison des méthodes précédentes (26) tout en laissant, de préférence, pour ne pas à avoir à ajouter ensuite de l'acide servant de catalyseur, au moins 0,02 équivalent H⁺ par mole de sucres. En chromatographie, on réunit donc la fraction de sucres à une partie de la fraction de sels. Sur résine d'échange d'ions, on passe de préférence successivement sur une résine échangeuse de cations et sur une résine échangeuse d'anions, la quantité de résine échangeuse d'anions pouvant être inférieure d'au moins 0,02 équivalents H⁺ à la stoechiométrie.
- à séparer le jus de sucres hydrolysés enzymatiquement en un jus de sucres neutres et en un jus de sucres anioniques. Cette opération (210) peut être réalisée par électrodialyse ou en fixant puis éluant les sucres anioniques sur une résine d'échange d'ions anionique.
- éventuellement à permuter le jus de sucres neutres sous forme acide par passage sur une résine échangeuse de cation ou par électrodialyse (28).
- si nécessaire à concentrer le sirop de sucres neutres par évaporation jusqu'à 30 à 95 % et plus particulièrement 60 à 80 % de matière sèche (29). Le sirop de sucres neutres débarrassé du marc solide présente une pureté en sucres généralement comprise entre 50 et 95 % par rapport à la matière sèche.
- à effectuer la glycosylation de l'alcool par le sirop de sucres neutres,
- à recueillir les glycosides d'alkyle tensioactifs.
- éventuellement à permuter le jus de sucres anioniques sous forme acide par passage sur une résine échangeuse de cation ou par électrodialyse (211).
- si nécessaire à concentrer le sirop de sucres anionique par évaporation jusqu'à 30 à 95 % et plus particulièrement 60 à 80 % de matière sèche (212). Le sirop de sucres anioniques débarrassé du marc solide présente une pureté en sucres anioniques généralement comprise entre 50 et 95 % par rapport à la matière sèche.
- à effectuer la glycosylation de l'alcool par le sirop de sucres anioniques,
- à recueillir les glycosides d'alkyle tensioactifs.

**Dans le cas où le substrat de départ contient des pectines selon une seconde variante** (figure 3), le procédé consiste :
- à mélanger les sous produits agricoles (31) tels que de les fruits, la pulpe de chicorée, les tubercules, le cynara à une solution aqueuse d'acide. On préfère l'acide sulfurique, l'acide chlorhydrique ou l'acide sulfosuccinique,
- à homogénéiser, puis à cuire le milieu à une température comprise entre 20 et 150 °C, plus particulièrement entre 80 et 140 °C, pendant une durée de 1 à 90 minutes (32),
- à presser le mélange à une pression de l'ordre de 1.10⁵ à 500.10⁵ Pascals et de préférence de l'ordre de 5.10⁵ à 60.10⁵ Pascals au moyen d'une presse à vis, de filtres presses ou avec tout autre équipement permettant d'exprimer un jus à partir de marc humide. Cette opération (33) permet d'isoler un jus de sucres (35) et un marc (34),
- éventuellement à diluer les marcs (34) avec au moins trois fois leur volume d'eau et les presser sur une presse identique à celle utilisée lors du premier pressage. Cette opération n'est pas indispensable mais permet d'augmenter le rendement d'extraction en sucres.
- à rassembler les jus des presses (jus de sucres (35))
- éventuellement à recycler une partie ou l'ensemble du jus brut acide (35) pour l'hydrolyse de nouveaux sous produits tels que les fruits, la pulpe de chicorée, les tubercules, le cynara, et de préférence avec une quantité de jus brut acide similaire à la première dilution. Plusieurs recyclages à la suite sont possibles et permettent d'hydrolyser les sous produits tels que les fruits, la pulpe de chicorée, les tubercules, le cynara, sans apport d'eau supplémentaire, seule la quantité d'acide est ajustée,
- à mettre en contact le jus de sucres (35) en présence de sels chélatants (36). On peut notamment utiliser des cations métalliques tels que l'hydroxyde d'aluminium, le sulfate d'aluminium, des cations alcalino-terreux tels que les hydroxydes de magnésium, de calcium, de strontium ou de baryum, les sulfates de magnésium, de calcium, de strontium ou de baryum, les chlorures de magnésium, de calcium, de strontium ou de baryum.
- si nécessaire à centrifuger le jus obtenu au moyen d'un équipement permettant d'obtenir des jus débarassés d'insolubles, tels qu'une décanteuse horizontale, un filtre presse, un décanteur statique, un filtre rotatif sous vide, des systèmes de filtration tangentielle, une centrifugeuse à assiettes auto-débourbeuse ou un filtre rotatif sous vide. On obtient alors le jus de sucres non chargés dépourvu d'insolubles et des boues (37). Les jus de sucres non chargés sont constitués de pentoses et d'hexoses et parfois de cétoses,
- éventuellement à déminéraliser le jus de sucres non-chargés, par chromatographie sur résine échangeuse d'ions ou par chromatographie d'exclusion à basse pression, par électrodialyse ou encore par une combinaison des méthodes précédentes (38) .
- éventuellement à permuter les jus de sucres non chargés par échange d'ions sur une résine échangeuse de cations ou par électodialyse (39).
- si nécessaire à concentrer le sirop de sucres non chargés par évaporation jusqu'à 30 à 95 % et plus particulièrement 60 à 80 % de matière sèche (310). Le sirop de sucres non chargés débarrassé du marc solide présente une pureté en sucres généralement comprise entre 50 et 95 % par rapport à la matière sèche.
- à effectuer la glycosylation de l'alcool par les sucres non chargés,
- à recueillir les glycosides d'alkyle tensioactifs.
- éventuellement à réaliser une hydrolyse enzymatique complémentaire des boues (37) afin d'obtenir des boues hydrolysées enzymatiquement. Cette opération consiste à mettre en contact les boues (37) issues de l'opération (36) en contact avec des pectinases pendant au moins 5 minutes, à une température comprise entre 10 et 60 °C et à un pH compris entre 3 et 6 (311).
- si nécessaire à centrifuger (312) les boues hydrolysées obtenues au moyen d'un équipement permettant d'obtenir des jus débarassés d'insolubles, tels qu'une décanteuse horizontale, un filtre presse, un décanteur statique, des systèmes de filtration tangentielle, ou une centrifugeuse à assiettes auto-débourbeuse. On obtient alors un liquide dépourvu d'insolubles et un solide (313),
- éventuellement à permuter le liquide par échange d'ions sur une résine échangeuse de cations ou par électodialyse (314).
- si nécessaire à concentrer le liquide par évaporation jusqu'à 30 à 95 % et plus particulièrement 60 à 80 % de matière sèche (315). Le liquide débarrassé du marc solide présente une pureté en sucres anioniques généralement comprise entre 50 et 95 % par rapport à la matière sèche.
- à effectuer la glycosylation de l'alcool par le liquide,
- à recueillir les glycosides d'alkyle tensioactifs.

Les mélanges de glycosides d'alkyle préparés selon le procédé de l'invention présentent des propriétés très intéressantes et trouvent notamment des applications dans les domaines de la détergence, de la cosmétique et de l'alimentaire.

Ces mélanges de glycosides d'alkyle sont de remarquables agents tensioactifs. Ils possèdent en particulier des propriétés de solubilisation, émulsionnantes, moussantes, mouillantes et dispersantes. Ils peuvent être utilisés comme détergents, notamment dans des compositions lessivielles et dans des compositions de lavage de la vaisselle, des sols et des vitres, comme adoucissants et comme adjuvants de détergents dans ces mêmes compositions aussi bien en poudre que liquide. Ils peuvent entrer également dans les compositions capillaires ou de dentifrice et être utiles en cosmétologie sous forme de pommades, de crèmes et de laits de beauté afin d'adoucir et de raffermir la peau en tant que tensioactif doux n'agressant ni la peau, ni les muqueuses. Leurs pouvoirs moussant et adoucissant peuvent être mis aussi à profit dans les compositions de bains moussants. Ils peuvent également entrer dans les formulations de produits alimentaires comme additifs d'huile et de graisse, dans les crèmes mayonnaises et les sauces. Dans l'industrie, ils peuvent servir d'agents pour effectuer des polymérisations en dispersion et en émulsion. Ce sont d'autre part des substances aptes à former des vésicules présentant des propriétés de cristal liquide.

Parmi ces propriétés, on doit tout particulièrement mentionner celle permettant d'abaisser la tension superficielle notamment de l'eau, le pouvoir moussant, le pouvoir émulsionnant et le pouvoir détergent.

Parmi les dérivés préparés selon le procédé de l'invention, on préfère pour leur bon pouvoir moussant ceux dans lesquels R¹ est alkyle et notamment alkyle ayant de 6 à 14 atomes de carbone et mieux encore de 8 à 12 et plus particulièrement 10 atomes de carbone dont le pouvoir moussant est supérieur aux produits classiques de référence. L'évaluation est réalisée selon la norme NFT 73404 qui consiste à faire s'écouler, selon un débit constant, 500 ml de solution de tensioactifs dans une éprouvette de 1000 ml graduée, thermostatée et contenant 50 ml de la même solution. La quantité de mousse générée par l'écoulement est estimée volumétriquement juste après la fin de l'écoulement et ensuite la stabilité du volume de mousse est évaluée en fonction du temps pendant 20 minutes. Les mélanges de pentosides de décyl-dodécyle présentent un pouvoir moussant supérieur à ceux des tensioactifs les plus couramment rencontrés tels que le dodécylbenzènesulfonate de sodium (SDBS), le dodécyl sulfate de sodium (SDS), le lauryléthersulfate de sodium à 2 moles d'oxyde d'éthylène (LES) ou la dodécylbétaïne.

Avec les mélanges préparés selon le procédé de l'invention, le volume initial de mousse est de 530 ml alors qu'il est inférieur à 450 ml pour les autres. Leur pouvoir moussant est également supérieur à ceux des polyglucosides d'alkyle (APG). Par ailleurs, ces composés présentent une très bonne stabilité dans le temps de la mousse, puisque le pourcentage de perte est généralement inférieur à 5 % après 20 minutes.

Les mélanges de glycosides d'alkyle préparés selon le procédé de l'invention abaissent très efficacement la tension superficielle de l'eau. Cette propriété a été déterminée par une technique usuelle de tensiométrie, en utilisant un tensiomètre Schlumberger Tensimat n° 3. La mesure est effectuée à 25°C. Le mobile de mesure est une lame de platine rectangulaire (25 mm x 5 mm). Quand R¹ a moins de 12 atomes de carbone, cet abaissement de la tension superficielle de l'eau est supérieur à celui observé avec les produits connus (SDBS, SDS, LES, APG). La mise en émulsion des salissures portées par du linge est ainsi rendue plus facile.

Pour leur pouvoir émulsionnant, on préfère tout particulièrement ceux dans lesquels R¹ est alkyle et notamment alkyle ayant de 8 à 22 atomes de carbone et mieux encore de 14 à 20.

Il convient enfin de souligner que les glycosides d'alkyle préparés selon le procédé de l'invention sont entièrement biodégradables et sans danger pour l'environnement, tout en présentant une bonne stabilité.

Les compositions réalisées à partir des mélanges préparés selon le procédé de l'invention peuvent se présenter sous différentes formes, notamment sous forme de détergents en poudre ou liquide, de lotions, moussantes ou non moussantes, d'émulsions de consistance liquide ou semi-liquide telles que des laits obtenus par dispersion d'une phase grasse dans une phase aqueuse ou inversement, de suspensions ou d'émulsions de consistance molle du type crèmes ou pommades, de gels ou encore de préparations solides telles que des sticks, des pains de nettoyage, des tampons imprégnés ou encore sous forme de masques hydratants.

Comme véhicule des compositions, on peut utiliser de l'eau, des solvants organiques compatibles avec une application topique tels que l'acétone, l'alcool isopropylique, les triglycérides d'acides gras en C₆-C₂₄, les éthers de glycol tels que les éthers d'alkyle inférieur de mono ou dialkylène glycol, dont le radical alkylène a de 2 à 4 atomes de carbone.

On peut également utiliser comme solvant des esters de polyalkylèneglycol et d'acide à chaîne courte en C₁-C₄, ou encore des silicones volatiles.

Les compositions réalisées à partir des mélanges préparés selon le procédé de l'invention peuvent également contenir des corps gras tels que des huiles naturelles ou synthétiques.

Les compositions réalisées à partir des mélanges préparés selon le procédé de l'invention peuvent également contenir des agents épaississants ou gélifiants tels que la cellulose ou des dérivés de cellulose. Les agents épaississants peuvent être également des polymères acryliques, des alginates, des gommes telles que la gomme de xanthane, de guar, de caroube ou la gomme arabique ou encore des polyéthylène glycols, des bentonites et des montmorillonites.

Les compositions réalisées à partir des mélanges préparés selon le procédé de l'invention peuvent également contenir des matières actives comme des agents hydratants ainsi que des adjuvants tels que des agents antioxydants, des agents conservateurs, des parfums, des colorants.

Les compositions réalisées à partir des mélanges préparés selon le procédé de l'invention peuvent également se présenter sous forme de solutions ou de dispersions contenant les pentosides d'alkyle sous forme vésiculaire, les vésicules pouvant alors servir d'agents d'encapsulation pour des ingrédients actifs lipophiles et/ou hydrophiles.

Une composition détergente en poudre réalisée à partir des mélanges préparés selon le procédé de l'invention comprend de 0,1 à 60 %, et de préférence de 10 à 30 % en poids d'une base détergente et de 99,9 à 40%, et de préférence de 90 à 70 % en poids d'adjuvants.

La base détergente peut être un dérivé ou un mélange de dérivés préparée selon le procédé de l'invention . Elle peut également être un mélange de un ou plusieurs dérivés préparés selon le procédé de l'invention avec un ou plusieurs tensioactifs classiques dans le domaine ; ces tensioactifs pouvant être anioniques, non ioniques, cationiques ou amphotères. La proportion de tensioactifs préparés selon le procédé de l'invention représente de 1 à 100 %, en poids, et de préférence de 50 à 100 % de l'ensemble de la charge en tensioactifs.

La composition détergente en poudre est utilisée à une concentration de 1 à 20 g/l, et de préférence entre 1 et 6 g/l. Le lavage est effectué dans une machine conventionnelle, entre 20 et 80°C, et de préférence entre 20 et 60°C, durant une période de 10 à 60 minutes.

Une composition détergente liquide réalisée à partir de dérivés préparés suivant le procédé de l'invention comprend de 0,1 à 60 %, et de préférence de 10 à 60 % en poids d'une base détergente de 99,9 à 40 % et de préférence de 90 à 70 % en poids d'adjuvants.

La base détergente liquide peut être un dérivé ou un mélange de dérivés préparés selon le procédé. Elle peut également être un mélange de un ou plusieurs dérivés préparés selon l'invention avec un ou plusieurs tensioactifs classiques dans le domaine.

La composition détergente liquide préparée à partir de dérivés synthétisés suivant le procédé de l'invention est utilisée en solution aqueuse à une concentration de 6 à 12 g/l, et à des températures de 40 à 70°C.

Les exemples suivants illustrent l'invention.

### Exemple 1

On met en contact 231 g de gros son d'orge à 95 % de matière sèche contenant par rapport à la matière sèche 30 % d'amidon, 15 % de protéines, 30 % de pentosanes et 770 g de solution d'acide sulfurique à 3 %. Le milieu réactionnel est homogénéisé dans un réacteur agité, puis porté à une température de 130 °C pendant 30 minutes. Après le choc thermique, le mélange est ramené à 60°C et l'on ajoute, 19 grammes de fleur de chaux à 90 % de matière sèche.
Le mélange est ensuite pressé sur une presse MARREL de laboratoire afin de séparer le marc 1 du jus 1. On recueille un jus et un marc à respectivement 17 % et 55 % de matière sèche.

Le jus 1 est ensuite déminéralisé par échange d'ions sur une résine cationique forte (IRA 200) et une résine anionique faible (IRA 94S). Le jus est ensuite concentré jusqu'à 72 % de matière sèche par évaporation de l'eau sous pression réduite ; sa composition est reportée dans le tableau suivant:

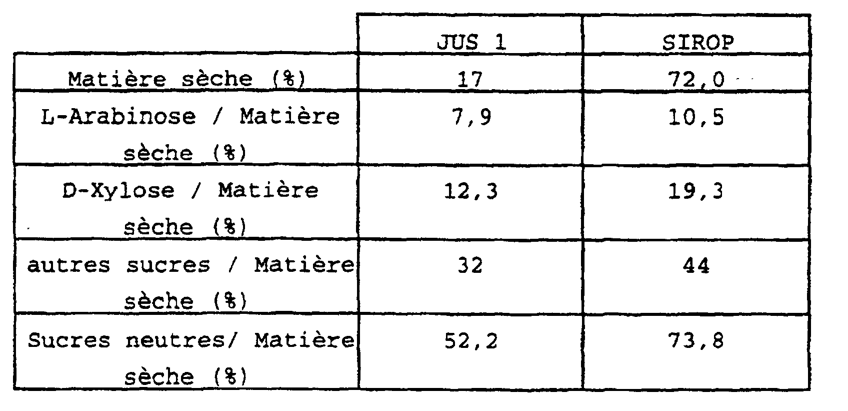

Nous récupérons 130 grammes de sirop ayant une pureté en sucres neutres de 73,8 %, les pentoses représentant 29,8 % de la matière sèche.

Ce sirop est ensuite ajouté goutte à goutte en 1 heure 30 à 125 g de *n*-butanol contenant 2,8 g d'acide sulfurique à une température de l'ordre de 102°C. L'eau est éliminée au cours de la réaction par distillation azéotropique du mélange eau/butanol. On ajoute ensuite le milieu réactionnel obtenu à 328 g d'alcool gras (dodécanol : 70%, tétradécanol : 30%) à une température de 90°C et en 2 heures. Le butanol est éliminé sous pression réduite en continu pendant l'addition. L'acidité du milieu est ensuite neutralisée par une solution aqueuse de soude à 35% jusqu'à pH 7 à 8. Les alcools gras en excès sont éliminés par évaporation au moyen d'un évaporateur à couche mince et pâles raclantes sous pression réduite (2 mb) à une température de 190°C. Le résidu (147 g) se présente sous la forme d'une pâte qui contient moins de 3% d'alcool gras résiduel. Celle-ci est mise en solution à 50% dans l'eau et est décolorée en présence d'eau oxygénée.

### Exemple 2

On met en contact 260 g de gros son de maïs séché à 92 % de matière sèche contenant par rapport à la matière sèche 30 % d'amidon, 8,5 % de protéines, 36 % de pentosanes et 700 g de solution d'acide sulfurique à 1,8 %. Le milieu réactionnel est homogénéisé dans un réacteur agité, puis porté à une température de 140 °C pendant 90 minutes. Après le choc thermique, le mélange est pressé sur une presse de laboratoire CARVER pour séparer le marc 1 du jus 1. On recueille un jus et un marc à respectivement 19,5 % et 45 % de matière sèche.
Le jus 1 est ensuite déminéralisé par échange d'ions sur une résine cationique forte (IRA 200) et une résine anionique faible (IRA 94 S) sa composition est reportée dans le tableau suivant :

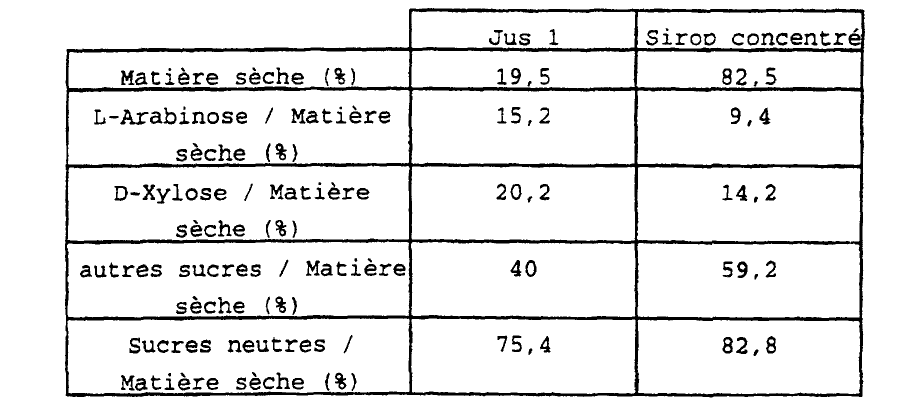

Ce jus est ensuite concentré jusqu'à une matière sèche de 82,5 % par évaporation de l'eau sous pression réduite. Nous récupérons 110 grammes de sirop ayant une pureté en sucres neutres de 82,8 %, les pentoses représentant 23,6 % de la matière sèche.

Ce sirop est ensuite ajouté goutte à goutte en 1 heure 30 à 130 g de *n*-butanol à 91% de matière sèche contenant 1,7 g d'acide sulfurique à une température de l'ordre de 102°C. L'eau est éliminée au cours de la réaction par distillation azéotropique du mélange eau/butanol. On ajoute ensuite le milieu réactionnel obtenu à 258 g d'alcool gras (décanol : 85%, dodécanol : 15 %) à une température de 90°C et en 2 heures. Le butanol est éliminé sous pression réduite en continu pendant l'addition. L'acidité du milieu est ensuite neutralisée par une solution aqueuse de soude à 35% jusqu'à pH 7 à 8. Les alcools gras en excès sont éliminés par évaporation au moyen d'un évaporateur à couche mince et pâles raclantes sous pression réduite (5 mb) à une température de 170 °C. Le résidu (136 g) se présente sous la forme d'une pâte qui contient moins de 3% d'alcool gras résiduel. Celle-ci est mise en solution à 50% dans l'eau et est décolorée en présence d'eau oxygénée.

### Exemple 3

110 g de sirop de sucres de l'exemple 2 sont ensuite ajoutés goutte à goutte en 3 heures à 260 g d'une coupe d'alcools gras (décanol : 85%, dodécanol : 15%) contenant 130 g de matière sèche de tensioactif de l'exemple 2 et 2,7 g d'acide sulfurique à une température de 100°C. L'eau est éliminée au cours de la réaction par évaporation sous pression réduite. L'acidité du milieu est ensuite neutralisée par une solution aqueuse de soude à 35% jusqu'à pH 7 à 8. Les alcools gras en excès sont éliminés par évaporation au moyen d'un évaporateur à couche mince et pâles raclantes sous pression réduite (5 mb) à une température de 170°C. Le résidu (127 g) se présente sous la forme d'une pâte qui contient moins de 3% d'alcool gras résiduel. Celle-ci est mise en solution à 50% dans l'eau et est décolorée en présence d'eau oxygénée.

### Exemple 4

110 g de sirop de sucres de l'exemple 2 sont ensuite ajoutés goutte à goutte en 1 heure 30 à 130 g de n-butanol à 91% de matière sèche contenant 2,7 g d'acide sulfurique à une température de l'ordre de 102°C. L'eau est éliminée au cours de la réaction par distillation azéotropique du mélange eau/butanol. On ajoute ensuite le milieu réactionnel obtenu à 412 g d'alcool gras (hexadécanol : 50%, octadécanol : 50%) à une température de 90°C et en 2 heures. Le butanol est éliminé sous pression réduite en continu pendant l'addition. L'acidité du milieu est ensuite neutralisée par une solution aqueuse de soude à 35% jusqu'à pH 7 à 8 à une température de 60°C. Le milieu réactionnel (510 g) est décoloré en présence d'eau oxygénée à 60°C et est ensuite refroidi. Il se présente sous la forme de paillettes, est constitué de 25 % de tensioactif et 70% d'alcool gras et peut être utilisé tel quel.

### Exemple 5

On met en contact 36 g de bagasse broyée séchée à 96 % de matière sèche contenant par rapport à la matière sèche 5 % de sucres neutres, 2,5 % de cendres, 18 % de pentosanes et 310 g de solution d'acide sulfurique à 0,6 %. Le milieu réactionnel est homogénéisé dans un réacteur agité, puis porté à une température de 120 °C pendant 60 minutes. Après le choc thermique, le mélange est pressé sur une presse de laboratoire MARREL pour séparer le marc 1 du jus 1. On recueille un jus et un marc à respectivement 3,93 % et 52 % de matière sèche.
Le jus 1 est ensuite déminéralisé par échange d'ions sur une résine cationique forte (IR 120) et une résine anionique faible (IRA 94S). Le jus est ensuite concentré jusqu'à 31 % de matière sèche par évaporation de l'eau sous pression réduite ; sa composition est reportée dans le tableau suivant :

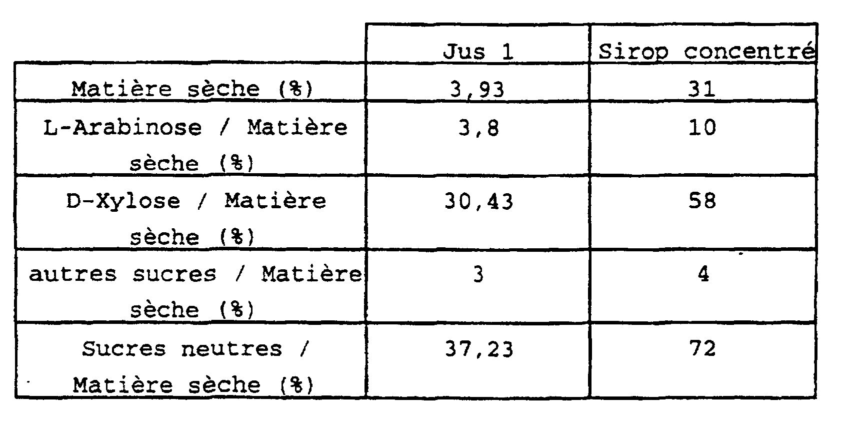

Ce jus est ensuite concentré jusqu'à une matière sèche de 31 % par évaporation de l'eau sous pression réduite. Nous récupérons 17 grammes de sirop ayant une pureté en sucres neutres de 72 %, les pentoses représentant 68 % de la matière sèche.

Ce jus est ensuite ajouté goutte à goutte en 1 heure à 7,7 g de *n*-butanol contenant 0,2 g d'acide sulfurique à une température de l'ordre de 102°C. L'eau est éliminée au cours de la réaction par distillation azéotropique du mélange eau/butanol. On ajoute ensuite le milieu réactionnel obtenu à 17 g d'alcool gras (décanol : 85%, dodécanol : 15%) à une température de 90°C et en 2 heures. Le butanol est éliminé sous pression réduite en continu pendant l'addition. L'acidité du milieu est ensuite neutralisée par une solution aqueuse de soude à 35% jusqu'à pH 7 à 8. Les alcools gras en excès sont éliminés par évaporation au moyen d'un évaporateur à couche mince et pâles raclantes sous pression réduite (5 mb) à une température de 170°C. Le résidu (8,5 g) se présente sous la forme d'une pâte qui contient moins de 2% d'alcool gras résiduel. Celle-ci est mise en solution à 50% dans l'eau et est décolorée en présence d'eau oxygénée.

### Exemple 6

On met en contact 500 g de marc de citrus à 31 % de matière sèche contenant par rapport à la matière sèche 20 % de pectines, 6,6 % de protéines, 40 % de sucres neutres et 449 g de solution d'acide sulfurique à 2,7 %. Le milieu réactionnel est homogénéisé dans un réacteur agité, puis porté à une température de 107 °C pendant 60 minutes. Le mélange est ensuite pressé sur une presse MARREL de laboratoire afin de séparer le marc 1 du jus 1. On recueille un jus et un marc à respectivement 10,8 % et 35 % de matière sèche.
Le jus 1 est ensuite, ramené à 60°C et l'on ajoute, 12 grammes de fleur de chaux à 90 % de matière sèche.
Le jus chaulé est ensuite centrifugé à 4080 g pendant 5 minutes afin de séparer le jus 2 du culot 1. On recueille un jus et un culot respectivement à 9 % et à 19 % de matière sèche.
Le jus 1 est ensuite déminéralisé par échange d'ions sur une résine cationique forte (IR 120) et une résine anionique faible (IRA 94S).
Le jus 2 est ensuite permuté sur une résine cationique forte et sa composition est reportée dans le tableau suivant :

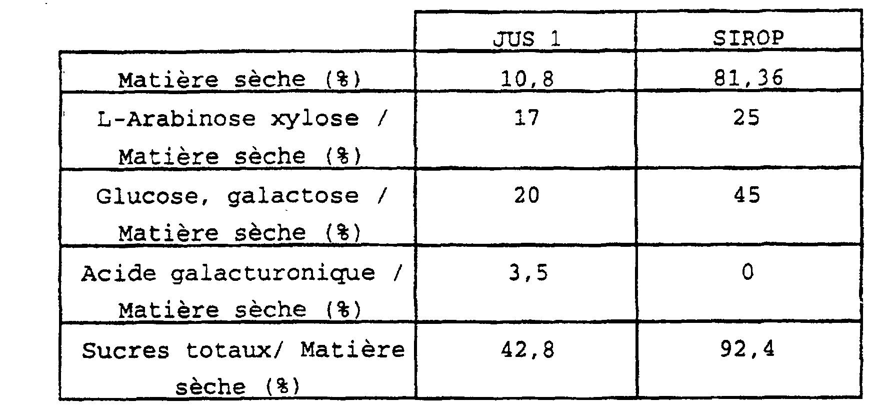

Nous récupérons 45 grammes de sirop ayant une pureté en sucres neutres de 92,4 %, les pentoses représentant 25 % de la matière sèche.

Ce sirop est ensuite ajouté goutte à goutte en 1 heure 30 à 47 g de *n*-butanol contenant 1,1 g d'acide sulfurique à une température de l'ordre de 102°C. L'eau est éliminée au cours de la réaction par distillation azéotropique du mélange eau/butanol. On ajoute ensuite le milieu réactionnel obtenu à 104 g d'alcool gras (décanol : 85%, dodécanol : 15%) à une température de 90°C et en 2 heures. Le butanol est éliminé sous pression réduite en continu pendant l'addition. L'acidité du milieu est ensuite neutralisée par une solution aqueuse de soude à 35% jusqu'à pH 7 à 8. Les alcools gras en excès sont éliminés par évaporation au moyen d'un évaporateur à couche mince et pâles raclantes sous pression réduite (5 mb) à une température de 160°C. Le résidu qui est constitué de tensioactifs non-ioniques, (57 g) se présente sous la forme d'une pâte qui contient moins de 2% d'alcool gras résiduel. Celle-ci est mise en solution à 50% dans l'eau et est décolorée en présence d'eau oxygénée.

Le culot est ensuite lavé avec 400 g d'eau puis centrifugé. On obtient un jus 3 et un culot 2.
Le culot 2 est redilué à 50 % puis hydrolysé à l'aide de pectinases (viscozyme L) dans un réacteur agité. La température d'hydrolyse est de 45 °C, la durée d'hydrolyse de 12 heures, le pH du jus de 4.
Le mélange est centrifugé à 4080 g pendant 5 minutes.
On obtient un hydrolysat et un culot 3, respectivement à 8,5 % et à 25 % de matière sèche.
L'hydrolysat est ensuite permuté sur une résine cationique forte (Amberlite 200) sous forme acide puis concentré jusqu'à 50 % de matière sèche par évaporation de l'eau sous pression réduite ; sa composition est reportée dans le tableau suivant :

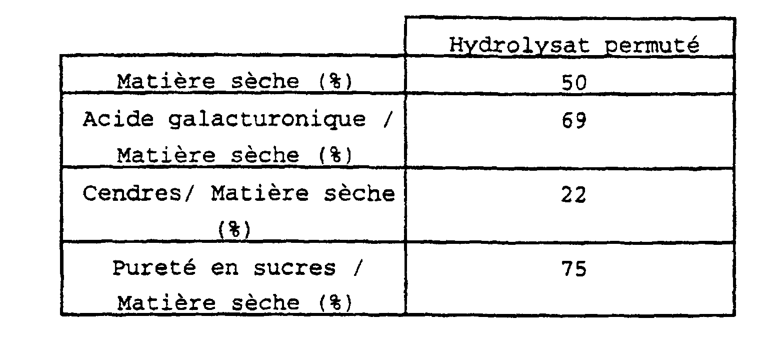

Nous récupérons 25 grammes de sirop ayant une pureté en acide galacturonique de 69 %.

Ce sirop est ensuite ajouté goutte à goutte en 1 heure à 15 g de *n*-butanol contenant 0,4 g d'acide sulfurique à une température de l'ordre de 102°C. L'eau est éliminée au cours de la réaction par distillation azéotropique du mélange eau/butanol. On ajoute ensuite le milieu réactionnel obtenu à 32 g d'alcool gras (décanol : 85%, dodécanol : 15%) à une température de 90°C et en 2 heures. Le butanol est éliminé sous pression réduite en continu pendant l'addition. On ajoute ensuite une solution aqueuse de soude à 35% afin de neutraliser le catalyseur acide et saponifier les tensioactifs neutres formés à partir de l'acide galacturonique. Le pH final est de l'ordre de 7 à 8. Les alcools gras en excès sont éliminés par évaporation au moyen d'un évaporateur à couche mince et pâles raclantes sous pression réduite (5 mb) à une température de 160°C. Le résidu qui est constitué majoritairement de tensioactifs anioniques (17 g) se présente sous la forme d'une pâte qui contient moins de 2% d'alcool gras résiduel. Celle-ci est mise en solution à 50% dans l'eau et est décolorée en présence d'eau oxygénée.

### Exemple 7

On met en contact 144 g de pelures de pommes à 27,45 % de matière sèche contenant par rapport à la matière sèche 16 % de pectines, 27 % de sucres neutres et 72 g de solution d'acide sulfurique à 5,4 %. Le milieu réactionnel est homogénéisé dans un réacteur agité, puis porté à une température de 107 °C pendant 90 minutes. Le mélange est ensuite pressé sur une presse MARREL de laboratoire afin de séparer le marc 1 du jus 1. Le jus 1 est ensuite ramené à 60°C et l'on ajoute, 12 ml de lait de chaux à 20 % de matière sèche.
Le jus chaulé est ensuite centrifugé à 4080 g pendant 5 minutes On recueille un jus 2 et un culot respectivement à 7,6 % et à 16 % de matière sèche.
Le jus 2 est ensuite déminéralisé par échange d'ions sur une résine cationique forte (IR 120) et une résine anionique faible (IRA 94S).
Le jus déminéralisé est ensuite permuté sur une résine cationique forte et concentré par évaporation sous pression réduite. Les compositions dus jus 2 et du sirop sont reportées dans le tableau suivant :

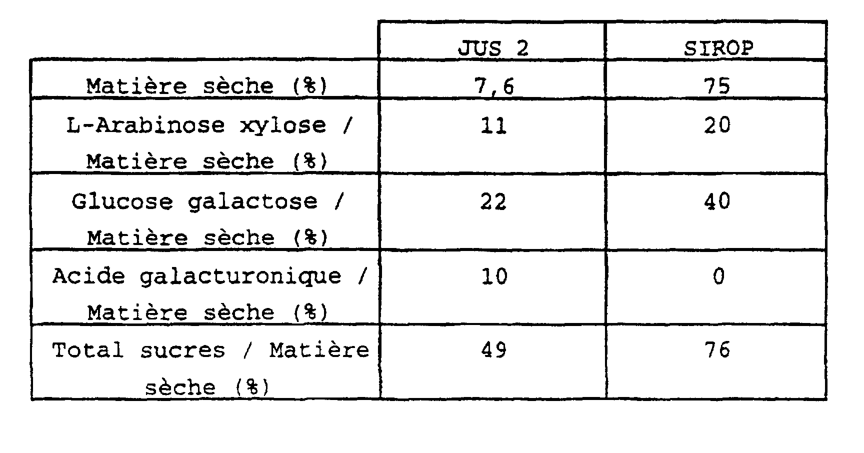

Nous récupérons 15 grammes de sirop ayant une pureté en sucres neutres de 78 %, les pentoses représentant 20 % de la matière sèche.

Ce sirop est ensuite ajouté goutte à goutte en 1 heure 30 à 15 g de *n*-butanol contenant 0,3 g d'acide sulfurique à une température de l'ordre de 102°C. L'eau est éliminée au cours de la réaction par distillation azéotropique du mélange eau/butanol. On ajoute ensuite le milieu réactionnel obtenu en à 29 g d'alcool gras (octanol : 50%, décanol : 50%) à une température de 90°C et en 2 heures. Le butanol est éliminé sous pression réduite en continu pendant l'addition. L'acidité du milieu est ensuite neutralisée par une solution aqueuse de soude à 35% jusqu'à pH 7 à 8. Les alcools gras en excès sont éliminés par évaporation au moyen d'un évaporateur à couche mince et pâles raclantes sous pression réduite (5 mb) à une température de 150 °C. Le résidu (12 g) se présente sous la forme d'une pâte qui contient moins de 2 % d'alcool gras résiduel. Celle-ci est mise en solution à 70 % dans l'eau et est décolorée en présence d'eau oxygénée.

Le culot est ensuite lavé avec 100 g d'eau puis centrifugé. On obtient un jus 3 et un culot 2.
Le culot 2 est redilué à 50 % puis hydrolysé à l'aide de pectinases (viscozyme L) dans un réacteur agité. La température d'hydrolyse est de 45 °C, la durée d'hydrolyse de 12 heures, le pH du jus de 4.
Le mélange est centrifugé à 4080 g pendant 5 minutes.
On obtient un hydrolysat et un culot 3, respectivement à 6,5 % et à 23 % de matière sèche.
L'hydrolysat est ensuite permuté sur une résine cationique forte (Amberlite 200) sous forme acide puis concentré jusqu'à 50 % de matière sèche par évaporation de l'eau sous pression réduite ; sa composition est reportée dans le tableau suivant :

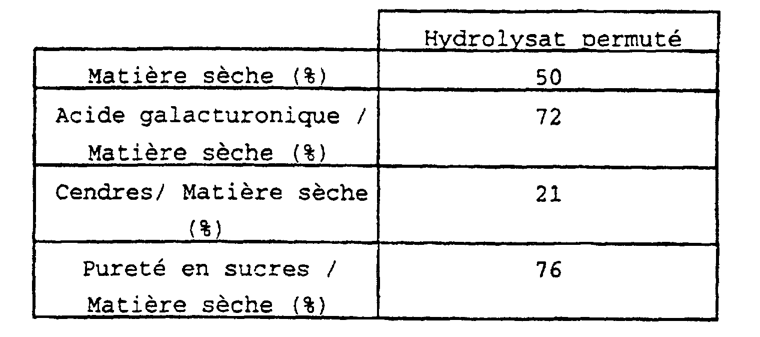

10 g de ce sirop sont ensuite ajoutés goutte à goutte en 1 heure à 6 g de *n*-butanol contenant 0,15 g d'acide sulfurique à une température de l'ordre de 102°C. L'eau est éliminée au cours de la réaction par distillation azéotropique du mélange eau/butanol. On ajoute ensuite le milieu réactionnel obtenu en à 12,5 g de décanol à une température de 90°C et en 2 heures. Le butanol est éliminé sous pression réduite en continu pendant l'addition. On ajoute ensuite une solution aqueuse de soude à 35% afin de neutraliser le catalyseur acide et saponifier les tensioactifs neutres formés à partir de l'acide galacturonique. Le pH final est de l'ordre de 7 à 8. L'alcool gras en excès est éliminé par évaporation au moyen d'un évaporateur à couche mince et pâles raclantes sous pression réduite (5 mb) à une température de 150°C. Le résidu qui est constitué majoritairement de tensioactifs anioniques (7 g) se présente sous la forme d'une pâte qui contient moins de 2% d'alcool gras résiduel. Celle-ci est mise en solution à 50% dans l'eau et est décolorée en présence d'eau oxygénée.

### Exemple 8

10 g du sirop d'acide galacturonique de l'exemple 7 sont ensuite ajoutés goutte à goutte en 1 heure à 6 g de *n*-butanol contenant 0,15 g d'acide sulfurique à une température de l'ordre de 102°C. L'eau est éliminée au cours de la réaction par distillation azéotropique du mélange eau/butanol. On ajoute ensuite le milieu réactionnel obtenu à 12,5 g de décanol à une température de 90°C et en 2 heures. Le bucanol est éliminé sous pression réduite en continu pendant l'addition. On ajoute ensuite de la triéthanolamine afin de neutraliser le catalyseur acide. Le pH final est de 7. L'alcool gras en excès est éliminé par évaporation au moyen d'un évaporateur à couche mince et pâles raclantes sous pression réduite (5 mb) à une température de 150°C. Le résidu qui est constitué majoritairement de tensioactifs non ioniques dérivés de l'acide galacturonique (9 g) se présente sous la forme d'une pâte qui contient moins de 2 % d'alcool gras résiduel. Celle-ci est décolorée à 60°C en présence d'eau oxygénée.

### Exemple 9

On met en contact 500 g un broyat de feuilles et de tiges du Cynara 507 à 90 % de matière sèche contenant par rapport à la matière sèche, 20 % de protéines, 25 % de pentosanes et 770 g de solution d'acide sulfurique à 0,25 %. Le milieu réactionnel est homogénéisé dans un réacteur agité, puis porté à une température de 120 °C pendant 60 minutes.
Le mélange est ensuite pressé sur une presse MARREL de laboratoire afin de séparer le marc 1 du jus 1. On recueille un jus et un marc à respectivement 6,9 % et 51,2 % de matière sèche.

Le jus 1 est ensuite déminéralisé sur une résine cationique forte (Amberlite 200) et une résine anionique faible (A 368S). Le jus est ensuite concentré jusqu'à 65 % de matière sèche par évaporation de l'eau sous pression réduite ;

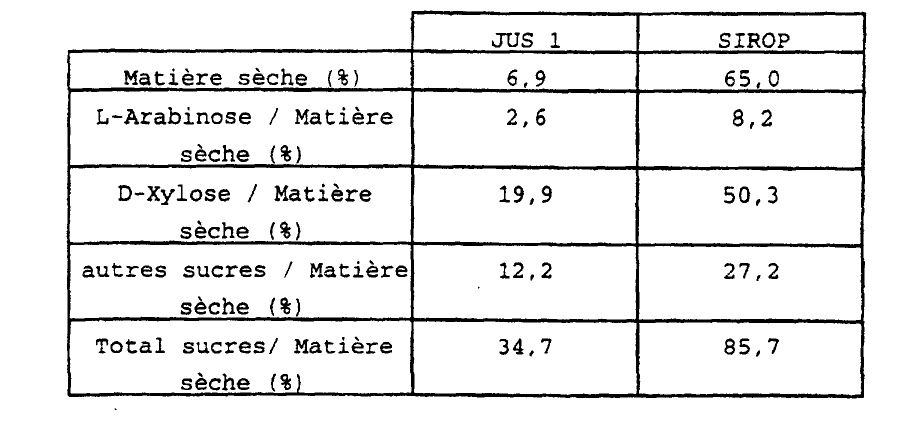

Nous récupérons 40 grammes de sirop ayant une pureté en sucres neutres de 85,7 %, les pentoses représentant 58,9 % de la matière sèche.

Ce sirop est ensuite ajouté goutte à goutte en 1 heure 30 à 36 g de *n*-butanol contenant 0,8 g d'acide sulfurique à une température de l'ordre de 102°C. L'eau est éliminée au cours de la réaction par distillation azéotropique du mélange eau/butanol. On ajoute ensuite le milieu réactionnel obtenu à 76,1 g de décanol à une température de 90°C et en 2 heures. Le butanol est éliminé sous pression réduite en continu pendant l'addition. L'acidité du milieu est ensuite neutralisée par une solution aqueuse de soude à 35% jusqu'à pH 7 à 8. L'alcool gras en excès est éliminé par évaporation au moyen d'un évaporateur à couche mince et pâles raclantes sous pression réduite (4 mb) à une température de 150°C. Le résidu (41 g) se présente sous la forme d'une pâte qui contient moins de 2% d'alcool gras résiduel. Celle-ci est mise en solution à 50% dans l'eau et est décolorée en présence d'eau oxygénée.

### Exemple 10

On met en contact 1000 g de marc de citrus à 31 % de matière sèche contenant par rapport à la matière sèche 20 % de pectines, 6,6 % de protéines, 40 % de sucres neutres et 898 g de solution d'acide sulfurique à 2,7 %. Le milieu réactionnel est homogénéisé dans un réacteur agité, puis porté à une température de 107 °C pendant 60 minutes. Le mélange est ensuite pressé sur une presse MARREL de laboratoire afin de séparer le marc 1 du jus 1. On recueille un jus et un marc à respectivement 10,8 % et 35 % de matière sèche.
Le jus 1 est ensuite déminéralisé successivement sur une résine cationique forte (Amberlite 200) puis sur un électrodialyseur (EUR 2B-20).

Le jus déminéralisé est ensuite hydrolysé à l'aide de pectinases (viscozyme L) dans un réacteur agité. La température d'hydrolyse est de 45 °C, la durée d'hydrolyse de 12 heures, le pH du jus de 4.
La composition de l'hydrolysat déminéralisé est reportée dans le tableau suivant :

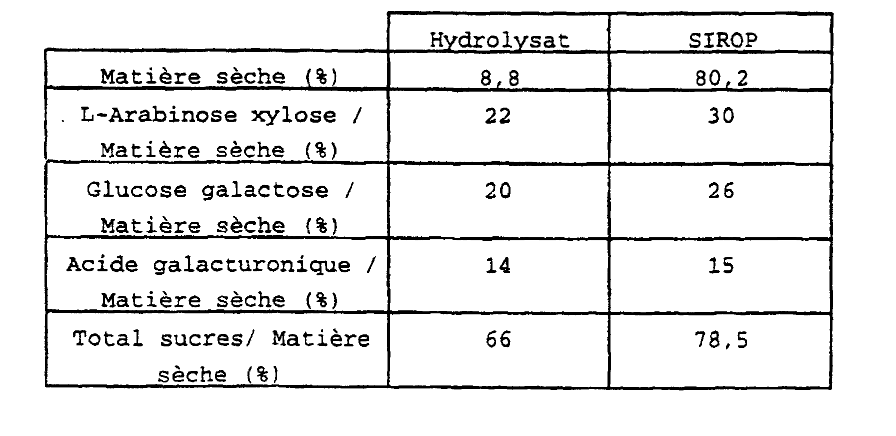

Nous récupérons 120 grammes de sirop ayant une pureté en sucres neutres de 63,5 %, les pentoses représentant 30 % de la matière sèche.

Ce sirop est ensuite ajouté goutte à goutte en 1 heure 30 à 121 g de *n*-butanol contenant 2,9 g d'acide sulfurique à une température de l'ordre de 102°C. L'eau est éliminée au cours de la réaction par distillation azéotropique du mélange eau/butanol. On ajoute ensuite le milieu réactionnel obtenu à 258 g de décanol à une température de 90°C et en 2 heures. Le butanol est éliminé sous pression réduite en continu pendant l'addition. On ajoute ensuite une solution aqueuse de soude à 35% afin de neutraliser le catalyseur acide et saponifier les tensioactifs neutres formés à partir de l'acide galacturonique. Le pH final est de l'ordre de 7 à 8. L'alcool gras en excès est éliminé par évaporation au moyen d'un évaporateur à couche mince et pâles raclantes sous pression réduite (5 mb) à une température de 150 °C. Le résidu qui est constitué d'un mélange de tensioactifs anioniques et non-ioniques (142 g) se présente sous la forme d'une pâte qui contient moins de 2% d'alcool gras résiduel. Celle-ci est mise en solution à 50% dans l'eau et est décolorée en présence d'eau oxygénée.

### Exemple 11

On met en contact 1000 g de marc de citrus à 31 % de matière sèche contenant par rapport à la matière sèche 20 % de pectines, 6,6 % de protéines, 40 % de sucres neutres et 898 g de solution d'acide sulfurique à 2,7 %. Le milieu réactionnel est homogénéisé dans un réacteur agité, puis porté à une température de 107 °C pendant 60 minutes. Le mélange est ensuite pressé sur une presse MARREL de laboratoire afin de séparer le marc 1 du jus 1. On recueille un jus et un marc à respectivement 10,8 % et 35 % de matière sèche.
Le jus 1 est ensuite déminéralisé successivement sur une résine cationique forte (Amberlite 200) puis sur un électrodialyseur (EUR 2B-20).

Le jus déminéralisé est ensuite hydrolysé à l'aide de pectinases (viscozyme L) dans un réacteur agité. La température d'hydrolyse est de 45 °C, la durée d'hydrolyse de 12 heures, le pH du jus de 4.
La composition de l'hydrolysat déminéralisé est reportée dans le tableau suivant :

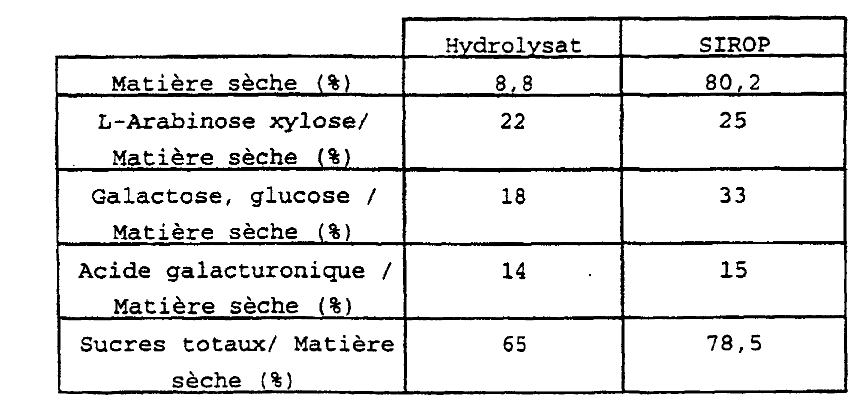

Nous récupérons 120 grammes de sirop ayant une pureté en sucres neutres de 63,5 %, les pentoses représentant 25 % de la matière sèche.

Ce sirop est ensuite ajouté goutte à goutte en 1 heure 30 à 121 g de *n*-butanol contenant 2,9 g d'acide sulfurique à une température de l'ordre de 102°C. L'eau est éliminée au cours de la réaction par distillation azéotropique du mélange eau/butanol. On ajoute ensuite le milieu réactionnel obtenu à 258 g de décanol à une température de 90°C et en 2 heures. Le butanol est éliminé sous pression réduite en continu pendant l'addition. On ajoute ensuite de la triéthanolamine afin de neutraliser le catalyseur acide. Le pH final est de 7. L'alcool gras en excès est éliminé par évaporation au moyen d'un évaporateur à couche mince et pâles raclantes sous pression réduite (5 mb) à une température de 150°C. Le résidu qui est constitué d'un mélange de tensioactifs non ioniques monocaténaires et bicaténaires (151 g) se présente sous la forme d'une pâte qui contient moins de 2 % d'alcool gras résiduel. Celle-ci est décolorée à 60°C en présence d'eau oxygénée.

### Exemple 12

On met en contact 885 g d'écorces d'oranges lavées, surpressées à 22 % de matière sèche contenant par rapport à la matière sèche 20 % de pectines, 6,6 % de protéines, 35 % de sucres neutres et 895 g d'eau. Le milieu réactionnel est homogénéisé dans un réacteur, puis le pH est contrôlé (pH 4).L'ensemble est porté à une température de 50 °C en réacteur agité et l'on ajoute 5 ml d'un cocktail enzymatique contenant essentiellement des pectinases et des cellulases (viscozyme 120 L) pendant 12 heures. Le mélange est ensuite séparé à l'aide d'une centrifugeuse de laboratoire à 4080 g pendant 5 minutes afin de séparer les boues 1 du jus 1. On recueille un jus et des boues respectivement à 6,8 % et 16,35 % de matière sèche.

Le jus 1 est ensuite permuté sous forme acide par échange d'ions sur une résine cationique forte (Amberlite 200).
Le jus 1 est ensuite electrodialysé (electrodialyseur de type EUR 5B-20) et l'on obtient un jus concentré et un jus dilué dont la composition est la suivante:

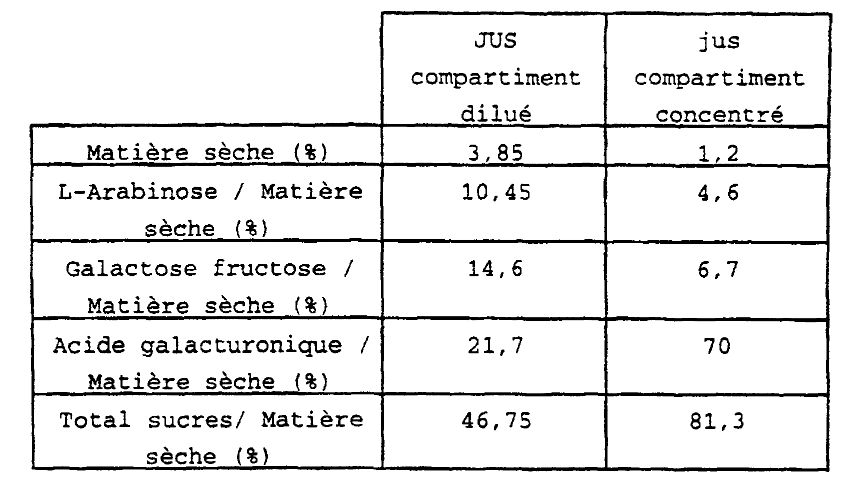

Nous récupérons le jus concentré et l'évaporons afin d'obtenir 10 grammes de sirop à 70 % de MS.Ce sirop possède une pureté en sucres totaux de 80 %, l'acide galacturonique représentant 70 % de la matière sèche.

Ce sirop est ensuite ajouté goutte à goutte en 1 heure 30 à 10,4 g de *n*-butanol contenant 0,25 g d'acide sulfurique à une température de l'ordre de 102°C. L'eau est éliminée au cours de la réaction par distillation azéotropique du mélange eau/butanol. On ajoute ensuite le milieu réactionnel obtenu à 23 g d'alcool gras (décanol : 85%, dodécanol : 15%) à une température de 90°C et en 2 heures. Le butanol est éliminé sous pression réduite en continu pendant l'addition. On ajoute ensuite une solution aqueuse de soude à 35% afin de neutraliser le catalyseur acide et saponifier les tensioactifs neutres formés à partir de l'acide galacturonique. Le pH final est de l'ordre de 7 à 8. Les alcools gras en excès sont éliminés par évaporation au moyen d'un évaporateur à couche mince et pâles raclantes sous pression réduite (5 mb) à une température de 160°C. Le résidu qui est constitué majoritairement de tensioactifs anioniques (7 g) se présente sous la forme d'une pâte qui contient moins de 2% d'alcool gras résiduel. Celle-ci est mise en solution à 50% dans l'eau et est décolorée en présence d'eau oxygénée.

### Réactivités relatives du L-arabinose de l'acide D-galacturonique et du D-glucose

Le sucre est mis en suspension dans 3 équivalents de décanol en présence de 3 % en masse par rapport au sucre d'acide sulfurique. Le milieu réactionnel est porté à 80°C et l'eau de la réaction est éliminée sous pression réduite 50 mb au fur et à mesure de sa formation. L'avancement de la réaction est suivi par CPG après silylation du milieu réactionnel. Les résultats sont énoncés dans la tableau suivant.

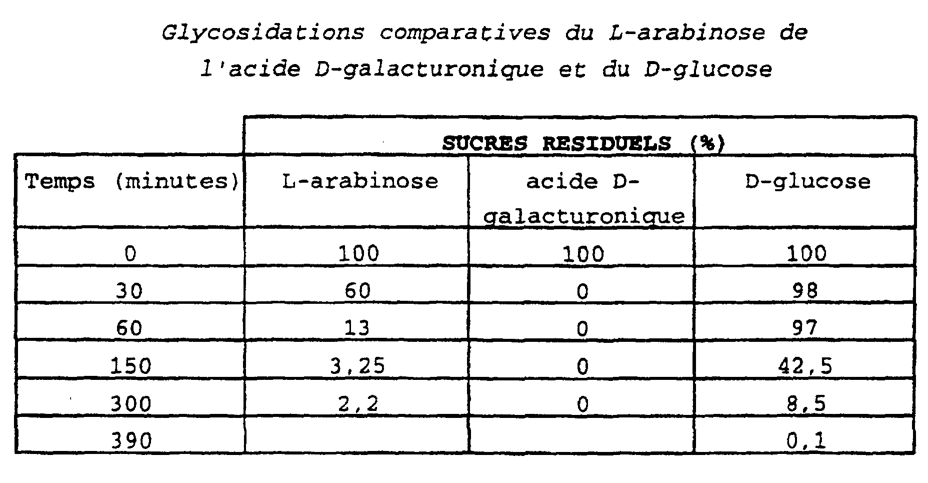

Dans les conditions de réaction définies précédemment, il est clair que le L-arabinose et l'acide D-galacturonique réagissent plus vite que le D-glucose pendant la glycosylation. Au bout de 30 minutes de réaction la totalité de l'acide D-galacturonique et 40 % de L-arabinose de départ ont été consommés alors que dans les mêmes conditions de réaction il reste au bout de 30 minutes, dans le cas du glucose, 98% de sucre résiduel.

### Exemple 14

### Réactivités du D-glucose en présence de L-arabinose

Le glucose ou le mélande de glucose et d'arabinose sont mis en suspension dans 3 équivalents de décanol en présence de 3 % en masse par rapport au sucre d'acide sulfurique. Le milieu réactionnel est porté à 80°C et l'eau de la réaction est éliminée sous pression réduite 50 mb au fur et à mesure de sa formation. L'avancement de la réaction est suivi par CPG après silylation du milieu réactionnel. Les résultats sont énoncés dans la tableau suivant.

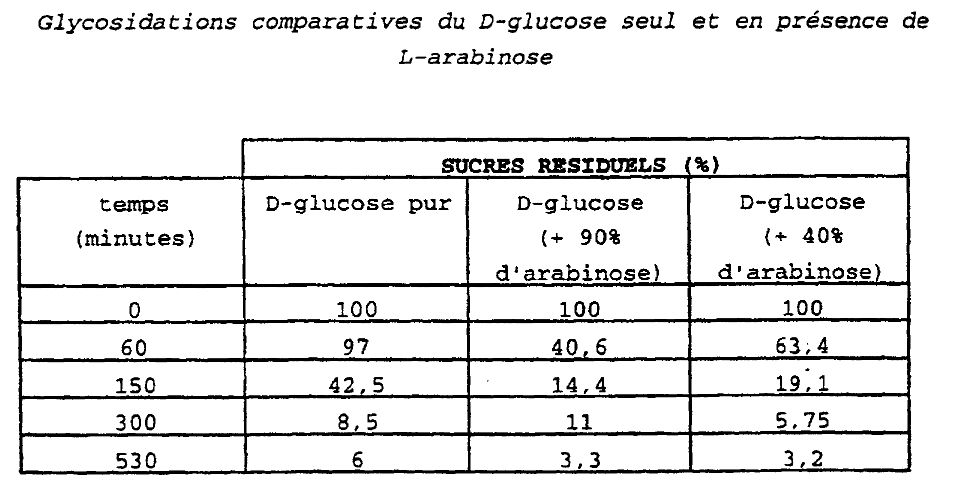

Dans les conditions de réaction définies précédemment,nous mettons en évidence en travaillant cette fois à partir d'un mélange de D-glucose et de L-arabinose une synergie entre ces deux sucres. Le D-glucose réagit en effet plus vite en présence d'arabinose que seul dans le milieu réactionnel. Au bout d'une heure de réaction, lorsque le glucose est seul dans le milieu réactionnel il reste 97% de celui-ci non modifié, alors que lorsqu'il est traité respectivement avec 90% et 40% de L-arabinose, il reste seulement 41% et 63 % de glucose résiduel.

## Revendications

1. Procédé de préparation d'agent tensioactif, **caractérisé en ce qu'**il consiste à mettre de la bagasse, des sous-produits du maïs, des sous-produits du sorgho, des sous-produits de l'orge, des sous-produits du riz, des fruits, de la pulpe de chicorée, des tubercules, du cynara en contact pendant au moins 5 secondes avec un agent d'hydrolyse choisi parmi une solution aqueuse acide entre 20 et 150°C et une composition enzymatique d'hydrolyse d'une matière végétale entre 20 et 90 °C pour obtenir un sirop de sucres contenant des pentoses, à débarrasser le sirop de sucres d'un marc solide pour obtenir un jus de sucres contenant des pentoses et à mettre le jus de sucres contenant des pentoses et, exempt de marc en contact avec un alcool ayant de 4 à 22 atomes de carbone à une température comprise entre 20 et 150°C jusqu'à obtention d'une solution de glycosides tensioactifs et à séparer le mélange des glycosides tensioactifs de cette solution.

2. Procédé suivant la revendication 1, **caractérisé en ce qu'**il consiste à mettre le jus de sucres en contact avec un alcool à une température comprise entre 40 et 90°C.

3. Procédé suivant l'une des revendications 1 à 2, **caractérisé en ce qu'**il consiste à utiliser comme fruit de la pulpe de citrus, du marc de pommes ou du marc de poires.

4. Procédé suivant l'une des revendications 1 à 2, **caractérisé en ce qu'**il consiste à utiliser comme tubercule de la pomme de terre ou des oignons.

5. Procédé suivant l'une des revendications 1 à 4, **caractérisé en ce qu'**il consiste quand le produit de départ contient des pectines à soumettre le jus de sucres, quand le subtrat est acidifié, à une hydrolyse enzymatique avant de le mettre en contact avec un alcool.

6. Procédé suivant la revendication 5, **caractérisé en ce qu'**il consiste après avoir effectué l'hydrolyse enzymatique qui donne un jus de sucres hydrolysé enzymatiquement à le séparer en un jus de sucres neutres et en un jus de sucres anioniques, à mettre séparément le jus de sucres neutres et éventuellement le jus de sucres anioniques en contact avec un alcool ayant de 4 à 22 atomes de carbone.

7. Procédé suivant la revendication 6, **caractérisé en ce qu'**il consiste à effectuer la séparation des sucres neutres des sucres anioniques par fixation des sucres anioniques sur des résines échangeuses d'ions de type anioniques puis à les récupérer, ou encore par électrodialyse.

8. Procédé suivant la revendication 1, **caractérisé en ce qu'**il consiste quand le produit de départ contient des pectines à mettre en contact le jus de sucres avec des sels chélatants de manière à obtenir un jus de sucres non chargés et des boues, à mettre le jus de sucres non chargés avec un alcool ayant de 4 à 22 atomes de carbone et éventuellement à mettre les boues en contact avec une ou plusieurs enzymes pour obtenir des boues hydrolysées et à les séparer en un liquide et un solide, à mettre le liquide en contact avec un alcool ayant de 4 à 22 atomes de carbone.

9. Procédé suivant l'une des revendications 1, 5 et 8, **caractérisé en ce qu'**il consiste à utiliser pour réaliser les hydrolyses enzymatiques l'une ou plusieurs des enzymes suivantes.Des pectinases, des hémicellulases, des cellulases, des β glucanases, des protéases, et des estérases.

10. Procédé suivant l'une des revendications 1, 5 et 8, **caractérisé en ce qu'**il consiste à effectuer l'hydrolyse enzymatique à une température comprise entre 20 et 90°C, pendant au moins 5 minutes et à un pH compris entre 3 et 6.

11. Procédé suivant l'une des revendications 1 à 8, **caractérisé en ce qu'**il consiste à effectuer sur le jus de sucres ayant éventuellement subi une hydrolyse enzymatique, ou bien sur le jus de sucres neutres, ou bien sur le jus de sucres anioniques, ou bien sur le jus de sucres non chargés ou bien sur le liquide tels qu'obtenus suivant le procédé de la revendication 8, une opération de permutation sous form acide avant de le mettre en contact avec un alcool.

12. Procédé suivant la revendications 11, **caractérisé en ce qu'**il consiste à effectuer l'opération de permutation en mettant en contact le jus avec une résine cationique forte ou par électrodialyse.

13. Procédé suivant la revendication 5, **caractérisé en ce qu'**il consiste après avoir effectué l'opération de mise en contact avec un alcool qui donne un mélange de tensioactifs neutres et à mettre en contact le mélange de tensioactifs neutres avec un agent de saponification.

14. Procédé suivant la revendication 13, **caractérisé en ce qu'**il consiste à utiliser comme agent de saponification de la soude.

## Claims

1. Process for preparing surfactant, **characterised in that** in comprises contacting cane trash, maize by-products, sorghum by-products, barley by-products, rice by-products, fruits, chicory pulp, tubers or Cynara for at least 5 seconds with a hydrolysing agent selected from an aqueous acid solution at between 20 and 150°C and an enzymatic hydrolysing composition of a plant material at between 20 and 90°C to obtain a sugar syrup comprising pentoses, freeing the sugar syrup from any solid residues so as to obtain a sugar liquor comprising pentoses and contacting the residue-free and pentoses comprising sugar liquor with a C₄₋₂₂-alcohol at a temperature of between 20 and 150°C, preferably between 30 and 110°C, until a solution of surfactant glycosides is obtained, and separating the surfactant glycosides from this solution.

2. Process according to claim 1, **characterised in that** it comprises contacting the sugar liquor with an alcohol at a temperature of between 40 and 90°C.

3. Process according to one of claims 1 and 2, **characterised in that** it comprises using citrus pulp, apple residue or pear residue as the fruit.

4. Process according to one of claims 1 and 2, **characterised in that** it comprises using potatoes or onions as the tuber.

5. Process according to one of claims 1 to 4, **characterised in that**, when the starting product contains pectins, it comprises subjecting the sugar liquor to enzymatic hydrolysis before bringing it into contact with an alcohol, when the substrate is acidified.

6. Process according to claim 5, **characterised in that**, after the enzymatic hydrolysis has been carried out, yielding an enzymatically hydrolysed sugar liquor, it comprises separating it into a neutral sugar liquor and a anionic sugar liquor, and separately bringing the neutral sugar liquor and optionally the anionic sugar liquor into contact with a C₄₋₂₂-alcohol.

7. Process according to claim 6, **characterised in that** it comprises separating the neutral sugars from the anionic sugars by fixing the anionic sugars on ion exchange resins of the anionic type and then recovering them, or by electrodialysis.

8. Process according to claim 1, **characterised in that**, when the starting product contains pectins, it comprises bringing the sugar liquors into contact with chelating salts so as to obtain an uncharged sugar liquor and sludges, bringing the uncharged sugar liquor into contact with a C₄₋ ₂₂-alcohol and optionally bringing the sludges into contact with one or more enzymes, in order to obtain hydrolysed sludges, and separating them into a liquid and a solid, and bringing the liquid into contact with a C₄₋₂₂-alcohol.

9. Process according to one of claims 1, 5, and 8, **characterised in that** it comprises using one or more of the following enzymes : pectinases, hemicellulases, cellulases, β-glucanases, proteases and esterases, for carrying out enzymatic hydrolysis.

10. Process according to one of claims 1, 5, and 8, **characterised in that** it comprises carrying out the enzymatic hydrolysis at a temperature between 20 and 90°C, for at least 5 minutes and at a pH of between 3 and 6.

11. Process according to one of claims 1 to 8, **characterised in that** it comprises carrying out an operation of permutation into acid form on the sugar liquor which may have undergone enzymatic hydrolysis, or on the neutral sugar liquor, or on the anionic sugar liquor, or on the uncharged sugar liquor or on the liquid as obtained by the process of claim 8, before bringing it into contact with an alcohol.

12. Process according to claim 11, **characterised in that** it comprises carrying out the operation of permutation by bringing the liquor into contact with a strong cationic resin or by electrodialysis.

13. Process according to claim 5, **characterised in that** it comprises, after carrying out the operation of contacting with an alcohol, which yields a mixture of neutral surfactants, bringing the mixture of neutral surfactants into contact with a saponification agent.

14. Process according to claim 13, **characterised in that** it comprises using sodium hydroxide as the saponification agent.

## Patentansprüche

1. Verfahren zur Herstellung eines oberflächenaktiven Agens, das sich dadurch kennzeichnet,
daß es daraus besteht, Bagasse, Nebenprodukte von Mais, Nebenprodukte von Sorghum, Nebenprodukte von Gerste, Nebenprodukte von Reis, Früchten, Chicorée-Pulpe, Knollen, Artischocken mindestens fünf Sekunden mit einem hydrolysierenden Agens in Kontakt zu bringen, ausgewählt aus einer sauren wässrigen Lösung zwischen 20 und 150°C und einer enzymatischen Hydrolysezusammensetzung eines pflanzlichen Materials zwischen 20 und 90°C; um einen Zuckersirup, der Pentosen enthält, zu erhalten, den Zuckersirup von der festen Schlempe zu lösen, um einen Zuckersirup mit einem Gehalt an Pentosen zu erhalten, und den Pentosen enthaltenden Zuckersaft ohne Schlempe mit einem Alkohol mit zwischen 4 bis 22 Kohlenstoffatomen bei einer Temperatur zwischen 20 und 150°C solange zu kontaktieren, bis eine Lösung von oberflächenaktiven Glycosiden erhalten wird und die Mischung der oberflächenaktiven Glycosiden von der Lösung abzutrennen.

2. Verfahren nach Anspruch 1, dadurch kennzeichnet, das es daraus besteht, den Zuckersaft mit einem Alkohol bei einer Temperatur zwischen 40 und 90°C in Kontakt zu bringen.

3. Verwendung und Verfahren nach einem der Ansprüche 1 bis 2, **dadurch gekennzeichnet, daß** es daraus besteht, als Frucht Citrusfrucht-Pulpe, Apfel-Schlempe oder Bimen-Schlempe zu verwenden.

4. Verwendung und Verfahren nach einem der Ansprüche 1 bis 2, **dadurch gekennzeichnet, daß** es darin besteht, als Knollen Kartoffeln oder Zwiebeln zu verwenden.

5. Verfahren nach einem der Ansprüche 1 bis 4, **dadurch gekennzeichnet, daß** es daraus besteht, daß, sobald das Ausgangsprodukt Pectine enthält, der Zuckersaft, nachdem das Substrat angesäuert worden ist, einer enzymatischen Hydrolyse unterworfen wird, bevor er in Kontakt mit einem Alkohol gebracht wird.

6. Verfahren nach Anspruch 5, **dadurch gekennzeichnet, daß** es daraus besteht, daß nach der enzymatischen Hydrolyse, die einen enzymatisch hydrolysierten Zuckersaft liefert um in einen Saft neutraler Zucker und einen Saft mit anionischen Zuckern zu trennen, getrennt der Saft der neutralen Zucker und gegebenenfalls den Saft der anionischen Zucker mit einem Alkohol mit 4 bis 22 Kohlenstoffatomen kontaktiert wird.

7. Verfahren nach Anspruch 6, **dadurch gekennzeichnet, daß** es darin besteht, daß die Auftrennung der neutralen Zucker und der anionischen Zucker durch Fixierung der anionischen Zucker auf lonenaustauscherharzen vom anionischen Typ bis zu ihrer Gewinnung oder auch durch Elektrodialyse bewirkt wird.

8. Verfahren nach Anspruch 1, **dadurch gekennzeichnet, daß** es darin besteht, daß dann, falls das Ausgangsprodukt Pektine aufweist, der Zuckersaft mit Chelat bildenden Salzen in Kontakt gebracht wird, so daß ein Saft von ungeladenen Zuckern und Abfall erhalten wird, der Saft der ungeladenen Zucker mit einem Alkohol mit 4 bis 22 Kohlenstoffatomen kontaktiert wird und gegebenenfalls der Abfall in Kontakt mit einem oder mehreren Enzymen in Kontakt gebracht wird, um hydrolysierten Abfall zu erhalten und diesen in eine Flüssigkeit und einen Feststoff zu trennen, um die Flüssigkeit in Kontakt mit einen Alkohol mit 4 bis 22 Kohlenstoffatomen zu bringen.

9. Verfahren nach einem der Ansprüche 1, 5 und 8, **dadurch gekennzeichnet, daß** es darin besteht, zur Realisation der enzymatischen Hydrolysen ein oder mehrere der nachfolgenden Enzyme zu verwenden: Pektinasen, Hemicellulasen, Cellulasen, β-Glucanasen, Proteasen und Esterasen.

10. Verfahren nach einem der Ansprüche 1, 5 und 8, **dadurch gekennzeichnet, daß** es darin besteht, daß die enzymatische Hydrolyse bei einer Temperatur zwischen 20 und 90°C während mindestens 5 Minuten bei einem pH zwischen 3 und 6 durchgeführt wird.

11. Verfahren nach einem der Ansprüche 1 bis 8, **dadurch gekennzeichnet, daß** es darin besteht, daß mit dem Zuckersaft, der gegebenenfalls einer enzymatischen Hydrolyse unterworfen wurde oder genauso mit dem Saft der neutralen Zucker oder genauso mit dem Saft der anionischen Zucker oder mit dem Saft der ungeladenen Zucker oder mit der Flüssigkeit, wie sie nach dem Verfahren der Ansprüche erhalten wird, eine Austauschoperation in die Saure Form durchgeführt wird, bevor dieser mit einem Alkohol in Kontakt gebracht wird.

12. Verfahren nach Anspruch 11, **dadurch gekennzeichnet, daß** es darin besteht, daß die Austauschoperation durchgeführt wird, indem der Saft mit einem starken Kationenaustauscherharz kontaktiert wird oder durch Elektrodialyse.

13. Verfahren nach Anspruch 5, **dadurch gekennzeichnet, daß** es darin besteht, daß nach der Durchführung des Betriebsschrittes des Kontaktierens mit einem Alkohol, der eine Mischung der neutralen Tenside liefert, die neutrale Tensidmischung mit einem Verseifungs-Agens kontaktiert wird.

14. Verfahren nach Anspruch 13, **dadurch gekennzeichnet, daß** es darin besteht, daß als Verseifungs-Agens Soda verwendet wird.
